# EUROPEAN PATENT APPLICATION

(11) **EP 1 956 096 A1**
(43) Date of publication of application: **13.08.2008**
(21) Application number: 07447011.3
(22) Date of filing: 12.02.2007
(51) Int. Cl.: C12Q 1/527, G01N 33/558, G01N 33/573

(54) **Method, device and kit for determining conditions related to a dysfunction of the renal proximal tubule**

(71) Applicant: Université Catholique De Louvain, 1348 Louvain-La-Neuve (BE)
(72) Inventor: The designation of the inventor has not yet been filed
(74) Representative: Brants, Johan P.E.

(57) **Abstract**

The present invention relates to method, device and kit for determining a condition related to dysfunction of the RPT in a subject comprising detecting the presence of type III Carbonic Anhydrase, CA-III, in a urine sample of said subject. It also relates to a method for monitoring the progress of a condition, and for determining the efficacy of a treatment.

## Description

### FIELD OF THE INVENTION

The present invention relates to the field of diagnosis. In particular, the invention provides a method for diagnosing conditions related to a dysfunction of the renal proximal tubule (RPT), also named renal Fanconi syndrome, by using a new urinary biomarker called type III Carbonic Anhydrase.
The invention further provides a diagnostic kit for diagnosing conditions related to a dysfunction of the RPT. In addition, the invention provides methods for identifying agents useful in the treatment of said conditions, and methods for monitoring the efficacy of a treatment for said conditions.

### BACKGROUND

The epithelial cells lining the renal proximal tubule (RPT) segment of the kidney play an essential role in reabsorbing ions, glucose, and amino acids from the primitive urine filtrated by the glomeruli. In particular, RPT cells avidly reabsorb several grams of albumin and low-molecular-weight (LMW) proteins that are daily filtered, through receptor-mediated endocytosis. This endocytic pathway, one of the most active of the body, involves two multiligand-binding receptors, megalin and cubilin, that are abundantly expressed at the brush border of RPT cells. Ligand binding and interactions between both receptors induce their internalization into coated vesicles at the apical membrane of RPT cells and their subsequent delivery to endosomes and lysosomes for ligand processing and receptor recycling. This endocytic trafficking is dependent on a progressive acidification from early to late endosomes and finally to lysosomes, a process that is driven by the vacuolar H⁺-ATPase (V-ATPase) complex coupled to a Cl⁻ conductance in order to dissipate the electrical gradient.

RPT dysfunction can be either inherited or acquired, for example after administering toxic compounds such as chemotherapy agents. A generalized RPT dysfunction, also named "renal Fanconi syndrome", is a severe condition associated with variable degrees of solute (phosphate, glucose, amino acids, bicarbonate, salt) wasting, polyuria, hypercalciuria, and LMW proteinuria, that can lead to growth retardation, osteomalacia, rickets, nephrocalcinosis, and renal failure.

Recent insights into the pathophysiology of rare inherited RPT dysfunctions pointed to the importance of receptor-mediated endocytosis in the process. Inactivating mutations in the *CLCN5* gene, which encodes the endosomal Cl-/H+ exchanger CIC-5, are associated with Dent's disease, an X-linked renal Fanconi syndrome characterized by LMW proteinuria and hypercalciuria, associated with glucosuria, amino-aciduria, phosphaturia, nephrocalcinosis, and nephrolithiasis. CIC-5 is primarily localized in the endosomes of RPT cells, where it co-distributes and is functionally linked with the V-ATPase. Genetic inactivation of Clcn5 in mouse causes renal tubular defects that mimic human Dent's disease, including severe RPT dysfunction with impaired endocytosis and trafficking defects. Likewise, the functional loss of cubilin in Imerslund-GrAsbeck disease, as well as the genetic inactivation of megalin in mouse, leads to defective RPT reabsorption with increased urinary excretion of LMW proteins.

The biochemical and metabolic outcomes of RPT cellular dysfunction associated with inherited renal Fanconi syndromes, as well as the potential adaptative mechanisms remain poorly understood. Recently, Wilmer *et al.* have reported an increased oxidative stress and altered redox status in RPT cells cultured from the urine of patients with cystinosis, the most frequent cause of inborn Fanconi syndrome. These observations suggest that RPT dysfunction may be associated with increased solicitation of cell oxidative defences.

The early diagnosis of RPT dysfunction is essential for early and efficient treatment. Nowadays the diagnosis of RPT dysfunction relies on blood and urine analyses. Urinanalyses mostly use solute markers which are freely filtered by the glomeruli and normally reabsorbed by RPT, such as glucose, β₂-microglobuline, uric acid, aminoacids, and phosphate. Thus far no specific markers of the RPT cell damage itself has been clearly described. Rarely, detection of RPT dysfunction requires a renal biopsy, so involving a surgical procedure.

In addition, there is an urgent need to allow selection of appropriate agent(s) for therapeutic and/or prophylactic treatments of RPT dysfunction. The prediction of drug responsiveness phenotype of one given patient to one given drug (efficiency, dosing, adverse effects, etc) remains poor, thereby hampering the therapy itself.

In view of the above, it is clear that there remains a need in the art for a method which enables early and sensitive detection of RPT diseases.

The present invention aims to provide a urinary biomarker which enables early and sensitive detection of RPT diseases, and which overcomes at least some of the above-mentioned problems of known makers.

In addition, the present invention aims to provide a method for diagnosis.

Another object of the present invention is to provide a method for choosing or monitoring the efficacy of various treatments for RPT disorders.

### SUMMARY OF THE INVENTION

One embodiment of the invention is a method for determining a condition related to dysfunction of the RPT in a subject comprising detecting the presence of type III Carbonic Anhydrase, CA-III, in a urine sample of said subject.

Another embodiment of the invention is a method as described above comprising the steps of:
(i) obtaining a urine sample from a subject;
(ii) measuring the concentration of CA-III in the sample;
(iii) comparing the concentration of CA-III in the sample with the concentration of CA-III in a healthy subject; and
(vi) determining a condition related to dysfunction of the PRT when the concentration of CA-III in the sample is at least 10% different from that measured in a sample from a healthy subject.

Another embodiment of the invention is a method as described above comprising the steps of:
(i) obtaining a urine sample from a subject;
(ii) measuring the concentration of CA-III in the sample;
(iii) determining a condition related to dysfunction of the PRT when detectable CA-III is present in the sample or concentration of CA-III in the sample is greater than or equal to a threshold concentration.

Another embodiment of the invention is a method as described above wherein said threshold value is between 1 pM and 1 mM.

Another embodiment of the invention is a method as described above comprising the steps of:
(i) obtaining a urine sample from a subject;
(ii) detecting the presence of CA-III the sample;
(vi) determining a condition related to dysfunction of the PRT when CA-III is detected in the sample.

Another embodiment of the invention is a method for monitoring the progress of a condition related to dysfunction of the PRT in a subject by monitoring the concentration of CA-III in two or more urine samples taken at different intervals.

Another embodiment of the invention is a method as described above, comprising the steps of:
(i) obtaining two or more urine samples from a subject, taken at different time intervals;
(ii) measuring the concentration of CA-III each sample;
(iii) determining the progress of a condition related to dysfunction of the PRT by comparing the concentrations of CA-III in the measured samples over time.

Another embodiment of the invention is a method for monitoring the efficacy of a treatment of a condition related to dysfunction of the PRT in a subject by detecting the level of CA-III in a urine sample taken before, after and optionally during treatment.

Another embodiment of the invention is a method as described above, comprising the steps of:
(i) obtaining a pre-administration urine sample from a subject prior to administration of the treatment;
(ii) measuring the concentration of CA-III in the pre-administration sample;
(iii) obtaining one or more post-administration urine samples from the subject;
(iv) measuring the concentration of CA-III the post-administration samples;
(v) comparing the concentration of CA-III in the pre-administration sample with the level of CA-III in the post-administration sample or samples; and
(vi) determining the efficacy of a treatment.

Another embodiment of the invention is a method as described above, further comprising the step of altering the treatment to improve the effect thereof.

Another embodiment of the invention is a method as described above, wherein the concentration or presence of CA-III in a sample is measured by using a CA-III specific probe.

Another embodiment of the invention is a method as described above, wherein said CA-III probe is an antibody directed against CA-III or a fragment thereof.

Another embodiment of the invention is a method as described above, wherein said antibody is a polyclonal antibody, monoclonal antibody, humanised or chimeric antibody, engineered antibody, or biologically functional antibody fragments sufficient for binding to CA-III.

Another embodiment of the invention is a method as described above, wherein said antibody is mouse monoclonal antibody clone 2CA-4.

Another embodiment of the invention is a method as described above wherein said CA-III human CA-III, a polypeptide having the sequence represent by SEQ ID NO: 1, or a fragment thereof.

Another embodiment of the invention is a method as described above, wherein the concentration or presence of CA-III is measured using any of biochemical assay, immunoassay, surface plasmon resonance, fluorescence resonance energy transfer, bioluminescence resonance energy transfer or quenching.

Another embodiment of the invention is a method as described above, wherein said condition is Fanconi syndrome or Dent's disease.

Another embodiment of the invention is a method as described above, wherein said condition is nephrotoxicity.

Another embodiment of the invention is a method as described above, wherein said arises from ingestion or infusion of heavy metals, chemotherapy agents, toxic drugs, poisons, pollutants, toxins or after injection with an iodinated contrast dye.

Another embodiment of the invention is a method as described above, wherein said condition is as a result of a physical renal injury.

Another embodiment of the invention is a method as described above, wherein said condition is renal or kidney failure or dysfunction.

Another embodiment of the invention is a method as described above, wherein said condition is acute renal failure include sepsis, shock, trauma, kidney stones, kidney infection, drug toxicity, poisons or toxins, or after injection with an iodinated contrast dye.

Another embodiment of the invention is a method as described above, wherein said condition is chronic renal failure, long-standing hypertension, diabetes, congestive heart failure, lupus, or sickle cell anemia.

Another embodiment of the invention is a method as described above, wherein said condition is inherited or acquired.

Another embodiment of the invention is a method as described above, further comprising detecting the presence of proteins and sugar in the urine.

Another embodiment of the invention is a use of CA-III as a urinary biomarker.

Another embodiment of the invention is a use of CA-III for diagnosing a condition related to dysfunction of the PRT in a subject.

Another embodiment of the invention is a device for determining a condition related to dysfunction of the PRT in a subject comprising means for determining the concentration and/or presence of CA-III in said urine sample.

Another embodiment of the invention is a device as described above, wherein said means comprise at least one CA-III specific probe.

Another embodiment of the invention is a device as described above, wherein said CA-III specific probe is as defined above.

Another embodiment of the invention is a device as described above, comprising a solid support whereby said CA-II is immobilised thereon.

Another embodiment of the invention is a device as described above, wherein said solid support comprises:
- fluid capillary properties:

- a distal (3) and proximal end (2),
- a sample application zone (4) in the vicinity of the proximal end (2),
- a reaction zone (5) distal to the sample application zone (4),
- a detection zone (6) distal to the reaction zone (5),
- where the reaction zone (5) is disposed with CA-III probe labelled with detection agent, that can migrate towards the distal end (3) in a flow of fluid by capillary action,
- where the detection zone (6) comprises said immobilised CA-III probe that can capture CA-III.

Another embodiment of the invention is a device as described above, housed in a cartridge (20) watertight against urine, having an opening (21) to provide access to the application zone (4) in proximal end (2), and another opening (22) to enable reading of detection zone (6) close to the distal end (3).

Another embodiment of the invention is a device as described above, wherein said cartridge (20) is disposed with a sensor code (23) for communicating with a reading device.

Another embodiment of the invention is a kit comprising a device as defined above and a urine sample container and/or control standards comprising CA-III.

The present invention relates to methods for the diagnostic and monitoring of RPT disorders, *i.e.* injuries or toxicities, and to kits for diagnosing renal toxicity. In particular, the invention relates to the use of a urinary biomarker to determine renal disorders even before the disorder is demonstrated by histopathology examination, and/or to help choosing or monitoring the efficacy of various treatments for renal disorders.

### DESCRIPTION OF THE FIGURES

**FIG. 1****:** Plan (A) and side view (B) of a test strip according to the invention.
**FIG 2****:** Plan view of a test cartridge according to the invention
**FIG 3****:** Real-time RT-PCR analyses of differentiation markers, such as PCNA, Ki67, cyclin E osteopontin, type I superoxide dismutase (SOD) and thioredoxin.
**FIG 4****:** Immunohistochemistry slides comparing PCNA- and K167-positive cells in CIC-5 deficient and non-deficient kidneys (left) and proliferation indices for the same.
**FIG 5A****:** Results of quantitative real-time RT-PCR to compare the mRNA expression of CAIII and CAII in Clcn5Y/- and Clcn5Y/+ kidneys.
**FIG 5B****:** Levels of CA-III mRMA expression in *Clcn5*^{*Y*/*-*} organs.
**FIG 5C****:** Immunoblotting analysis showing absence of antibody cross-reactivity between the two isozymes of CA-III.
**FIG 5D****:** Immunoblotting analysis showing levels of CA-III and CA-Ilin 12-week-old CIC-5 deficient and non-deficient kidneys.
**FIG 5E****:** Optical density analyses of the results obtained in 5D.
**FIG. 6****:** Immunoblotting analyses indicating a specific excretion of CAIII in the urine of the Clcn5Y/- compared with Clcn5Y/+.
**FIG. 7A to E:** Immunohistochemistry slides comparing signal for CAIII in various samples.
**FIG 8A to F:** Immunogold analyses indicating CAIII distribution.
**FIG. 9A** **and C:** Levels of CAIII for both mRNA and protein levels in kidney samples with Dent's disease in comparison to 4 end-stage kidney samples taken as controls.
FIG. **9B****:** Real-time RT-PCR studies of mRNA expression in Dent kidney.
**FIG. 9C****:** lmmunohistochemistry slides indicating the expression of CA-III in PT cells, identified by co-staining with the water channel aquaporin-1 (panel D).
**FIG. 10****:** mRNA expression levels (A) of CAII and CA-III in HK-2 cells after incubation with H₂O₂. Immunoblotting analyses (B) showing an early and stable induction of CAIII from 6 hours postincubation of HK-2 cells with H₂O₂.

### DETAILED DESCRIPTION OF THE INVENTION

Unless defined otherwise, all technical and scientific terms used herein have the same meaning as is commonly understood by one of skill in the art. All publications referenced herein are incorporated by reference thereto. All United States patents and patent applications referenced herein are incorporated by reference herein in their entirety including the drawings.

The articles "a" and "an" are used herein to refer to one or to more than one, i.e. to at least one of the grammatical object of the article. By way of example, "a sample" means one sample or more than one sample.

Throughout this application, the term "about" is used to indicate that a value includes the standard deviation of error for the device or method being employed to determine the value.

The recitation of numerical ranges by endpoints includes all integer numbers and, where appropriate, fractions subsumed within that range (e.g. 1 to 5 can include 1, 2, 3, 4 when referring to, for example, a number of samples, and can also include 1.5, 2, 2.75 and 3.80, when referring to, for example, measurements). The recitation of end points also includes the end point values themselves (e.g. from 1.0 to 5.0 includes both 1.0 and 5.0).

The present invention relates to the finding by the inventors that a level of carbonic anhydrase III (CA-III) is locally modulated when there is a dysfunction of the RPT cells, and that the level of urinary excretion of CA-III is directly linked to RPT damage. In other words, a dysfunction of the RPT can be detected by measuring the level of CA-III in the urine. This technique relies on a specific RPT marker and avoids the need to take a biopsy of the kidney or RPT, which may be required to obtain a reliable diagnosis. Surprisingly the inventors have found that CA-III is excreted into the urine, and, therefore, crosses the blood/urine barrier unlike many other disease markers which are filtered by the glomerular membrane before eventual RPT reabsorption. Furthermore, the level of CA-III production in dysfunctional RPT corresponds to the level detected in urine. This means elevated or reduced local CA-III levels are not distorted by any effect of storage in the bladder or modification by the urine. Additionally, they have found that CA-III in urine can be detected by specific binding assays *i.e.* there is little or no modification by the urine on CA-III at the molecular level.

The inventors have found that this marker is extremely sensitive and allows the early diagnosis of RPT dysfunction such that a suitable therapy can be initiated at an early stage in the course of a disease. It also permits exquisite monitoring of a progress of a condition disease, and evaluation of its treatment.

### Conditions related to dysfunction of the RPT

A condition related to a dysfunction of the RPT is any that gives rise to the abnormal functioning of the epithelial cells lining the RPT, also called renal Fanconi syndromes.

A dysfunction of the RPT may be inherited or acquired.

A dysfunction of the RPT can also be a result of toxicity (nephrotoxicity) and can arise from ingestion or infusion of heavy metals, chemotherapy agents, toxic drugs, poisons, pollutants, toxins or after injection with an iodinated contrast dye (adverse effect) etc.

A dysfunction of the RPT can also be a result of a renal injury.

A dysfunction of the RPT can also be a result of a renal or kidney failure or dysfunction either sudden (acute) or slowly declining over time (chronic). Conditions which give rise to acute renal failure include sepsis (infection), shock, trauma, kidney stones, kidney infection, drug toxicity, poisons or toxins, or after injection with an iodinated contrast dye (adverse effect); conditions which give rise to chronic renal failure include long-standing hypertension, diabetes, congestive heart failure, lupus, or sickle cell anemia. Both forms of renal failure result in a life-threatening metabolic derangement.

### Urine sample

The urine sample as used herein is generally unprocessed urine. However, the invention includes urine to which common stabilizing additives such as anti-bacterial-growth agents or protein stabilizing agents have been added, as well as urine sediments and supernatant obtained by centrifuging urine. The volume of urine required to perform the assay will depend on the technique used to assay the amount of CA-III. The skilled person can readily perform tests to determine the sensitivity of the assay using control sample of CA-III, and adapt the volume of urine required accordingly. The subject may provide a urine sample in a regular urine sample bottle which typically holds between 25 to 100 ml urine.

### Carbonic anhydrase III

The CA-III refers to full length human CA-III. According to an aspect of the invention, CA-III is a polypeptide having the sequence represent by SEQ ID NO: 1 in Table 1.

**Table 1: Amino acid sequence of CA-III according to the invention**

| |
|---|
| SEQ ID NO: 1, amino acid sequence of human CA-III (Accession no: NP_005172) |
| MAKEWGYASH NGPDHWHELF PNAKGENQSP VELHTKDIRH DPSLQPWSVS YDGGSAKTIL NNGKTCRVVF DDTYDRSMLR GGPLPGPYRL RQFHLHWGSS DDHGSEHTVD GVKYAAELHL VHWNPKYNTF KEALKQRDGI AVIGIFLKIG HENGEFQIFL DALDKIKTKG KEAPFTKFDP SCLFPACRDY WTYQGSFTTP PCEECIVWLL LKEPMTVSSD QMAKLRSLLS SAENEPPVPL VSNWRPPQPI NNRVVRASFK |

CA-III also refers to a fragment of CA-III which has a unique property, allowing identification of the fragment as a fragment of CA-III. A unique property may be, for example, a unique sequence or unique reactivity with binding agent such as an antibody or probe.

According to one embodiment of the invention, a fragment of CA-III comprises one or more contiguous deletions from the C- or N-terminal end, or both. The number of deletions may be equal to or less than 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50 amino acids. Preferably the number of deletions is between 1 and 30 amino acids.

### Detecting presence of a condition

One embodiment of the present invention is a method for diagnosing a condition related to dysfunction of the RPT in a subject by detecting the presence or level of CA-III in a urine sample. The level may be compared with that of healthy subjects.

In a preferred embodiment, the present invention provides a method for determining a condition related to dysfunction of the RPT in a subject comprising the steps of:
(i) obtaining a urine sample from a subject;
(ii) measuring the concentration of CA-III in the sample;
(iii) comparing the concentration of CA-III in the sample to the concentration of CA-III in a healthy subject sample; and
(vi) determining a condition related to dysfunction of the PRT when the concentration of CA-III in the sample is at least 10% different from that measured in a sample from a healthy subject.

One embodiment of the present invention, a concentration of CA-III in a sample that is at least 10% (e.g. at least 20%, 30%, 40%, 50%, 60%, 70%, 80% or 90%) different from that measured in a sample from a healthy subject, identifies the subject as having a dysfunction of the RPT. The concentration of CA-III in the sample may be higher or lower than that in a healthy subject to indicate a dysfunction; preferably it is higher.

According to another preferred embodiment, the present invention provides a method for determining a condition related to dysfunction of the RPT in a subject comprising the steps of:
(i) obtaining a urine sample from a subject;
(ii) measuring the concentration of CA-III in the sample;
(iii) determining a condition related to dysfunction of the PRT when detectable CA-III is present in the sample or concentration of CA-III in the sample is greater than or equal to a threshold concentration.

The threshold concentration can be extremely low as the inventors found that no detectable CA-III is present in healthy subjects. According to one aspect of the invention, the threshold concentration is 1 pM, 5 pM, 10 pM, 50 pM, 100 pM, 500 pM, 1 nM, 5 nM, 10 nM, 50 nM, 100 nM, 500 nM, 1*µ*M, 5 µM, 10 µM, 50 µM, 100 µM, 500 µM, 1mM, 5 mM, 10 mM, 50 mM, 100 mM, 500 mM; the concentration of CA-III in the sample a value in the range between any two of the aforementioned values; preferably it is between 1 pM and 1 mM.

The inventors have found that a healthy subject may have no detectable CA-III in their urine. Therefore, a qualitative (yes/no) rather than a quantitative (concentration) measure of CA-III may only be necessary to determine the presence of a condition.

In a preferred embodiment, the present invention provides a method for determining a condition related to dysfunction of the RPT in a subject comprising the steps of:
(i) obtaining a urine sample from a subject;
(ii) detecting the presence of CA-III in the sample;
(vi) determining a condition related to dysfunction of the RPT when CA-III is detected in the sample.

The presence of CA-III in a sample, and/or concentration thereof can be determined using the binding assays described below.

### Monitoring progress of a disease

Another embodiment of the present invention is a method for monitoring the progress of a condition related to dysfunction of the RPT in a subject by monitoring the concentration of CA-III in two or more urine samples taken over time intervals. The level may be compared with that of healthy subject.

The monitoring generally entails measuring the level of CA-III in urine sample from a subject, which sample is taken at regular periods e.g. over the course of a number of days, weeks or months. The level of CA-III in the urine sample over time can give an indication of whether a condition is improving, worsening or has stabilized.

In a preferred embodiment, the present invention provides a method for monitoring the progress of a condition related to dysfunction of the RPT in a subject comprising the steps of:
(i) obtaining two or more urine samples from a subject, taken at different time intervals;
(ii) measuring the concentration of CA-III in each sample;
(iii) determining the progress of a condition related to dysfunction of the RPT by comparing the concentrations of CA-III in the measured samples over time.

The time interval may be any which can give can give rise to a detectable change in the case of disease progression or retardation. The time interval can depend on the sensitivity of the measurement step. For example, a highly sensitive technique, with little background signals, might reveal small changes in the concentrations of CA-III in the sample; consequently the time interval between sample can be short e.g. between 1 to 7 days. A less sensitive technique, on the other hand, would not reveal small changes, so necessitating larger time interval between samples e.g. between 1 to 3 weeks. According to one embodiment of the invention, the time interval between samples can be less than or equal to 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50 days or a value in the range between any two of the aforementioned values. Preferably the time interval is between 1 to 10 days.

The concentration of CA-III in a sample can be determined using the binding assays described below.

### Monitoring the efficacy of a treatment

Another embodiment of the present invention is a method for monitoring the efficacy of a treatment of a condition related to dysfunction of the RPT in a subject by detecting the level of CA-III in a urine sample taken before, after and optionally during treatment. A reduction in the level of CA-III will indicate an effective treatment.

Where the treatment comprises the administration of an agents (e.g., drug compounds, an agonist, antagonist, peptidomimetic, protein, peptide, nucleic acid, small molecule, or other drug candidate), the invention can be advantageously applied in clinical trials. For example, the effectiveness of an agent to affect the levels of CA-III can be monitored in clinical trials of subjects receiving treatment for RPT dysfunction. Furthermore, the treatment can be altered according to the efficacy of the treatment

In a preferred embodiment, the present invention provides a method for monitoring the effectiveness of treatment of a subject with an agent (e.g.) comprising the steps of:
(i) obtaining a pre-administration urine sample from a subject prior to administration of the agent;
(ii) measuring the concentration of CA-III in the pre-administration sample;
(iii) obtaining one or more post-administration urine samples from the subject;
(iv) measuring the concentration of CA-III in the post-administration samples;
(v) comparing the concentration of CA-III in the pre-administration sample with the level of CA-III in the post-administration sample or samples; and
(vi) determining the efficacy of a treatment.

After determining the efficacy of a treatment, the treatment can be changed, for example, to improve the effect. This might entail altering an agent and/or administration thereof to the subject accordingly. For example, modified administration of the agent can be desirable to decrease the level of CA-III to lower levels than detected, *i.e.,* to increase the effectiveness of the agent. Alternatively, increased/decreased administration of the agent can be desirable to increase/decrease the effectiveness of the agent, respectively.

Another particular aspect of the present invention, a method is provided for both prophylactic and therapeutic methods of treating a subject having, or at risk of having, a kidney disorder or renal toxicity. Administration of a prophylactic agent can occur prior to the manifestation of symptoms characteristic of the kidney disorder, such that development of the kidney disorder is prevented or delayed in its progression. Examples of suitable therapeutic agents include, but are not limited to, antisense nucleotides, ribozymes, double-stranded RNAs, ligands, small molecules and antagonists.
The concentration of CA-III in a sample can be determined using the binding assays described below.

### Binding assays for detecting level of CA-III

One embodiment of the present invention, the presence and/or concentration of CA-III in a sample is detected by using a CA-III probe specific for CA-III. Typically, CA-III probe and CA-III form a complex which causes a physical change (e.g. colour change, change in polarization) compared with the uncomplexed forms, which physical change can be detected. The magnitude of the change is usually in proportion to the quantity of complex. When the reaction is calibrated with standard concentrations of CA-Ill, the quantity of CA-III can be calculated by comparing the change with a standard. It is not always necessary to employ standard controls, for example, if, for example, the concentration of CA-III probe is known, and tight binding is assumed, it can be possible to calculate the concentration of CA-III. When a only qualitative result is needed, standard concentration controls may not be needed

As used herein, the binding between CA-III and CA-III probe refers to their physical association. The binding is generally specific, meaning it occurs with a Kd of 1 mM or less, generally in the range of 100 nM to 10 pM. For example, binding is specific if the Kd is 100 nM, 50 nM, 10 nM, 1 nM, 950 pM, 900 pM, 850 pM, 800 pM, 750 pM, 700 pM, 650 pM, 600 pM, 550 pM, 500 pM, 450 pM, 350 pM, 300 pM, 250 pM, 200 pM, 150 pM, 100 pM, 75 pM, 50 pM, 25 pM, 10 pM or less. Being specific can also mean it is unique, *i.e.* there is no cross-reactivity between the CA-III probe any other substance besides CA-III and a fragment thereof.

The measuring is typically performed under conditions permitting the binding between CA-III and a CA-III probe; this refers to conditions of, for example, temperature, salt concentration, pH and protein concentration under which binding will arise. Exact binding conditions will vary depending upon the nature of the assay, for example, whether the assay uses pure probe or only partially purified probe. Temperatures for binding can vary from 15 deg C to 37 deg C, but will preferably be between room temperature and about 30 deg C.

One embodiment of the present invention, a concentration of CA-III in a sample that is at least 10% (e.g., at least 20%, 30%, 40%, 50%, 60%, 70%, 80% or 90%) different from that measured in a sample from a healthy subject, identifies the subject as having a dysfunction of the RPT. The concentration of CA-III in the sample may be higher or lower than that in a healthy subject to indicate a dysfunction; preferably it will be higher.

The measuring may be performed using any method known in the art. Preferably, the method selected from biochemical assay (e.g., solid phase assay), surface plasmon resonance, fluorescence resonance energy transfer (FRET), bioluminescence resonance energy transfer (BRET), fluorescence quenching, and fluorescence polarisation. Such techniques are well known and are described as follows.

### Biochemical assays

Biochemical assays for determining the binding between two components generally rely on the immobilisation of one binding component, for example, on a membrane or other solid support, and exposure to the second binding component. After washing away excess of the second binding component, bound complex is detected by, for example, immunoassay, or by using labelled components (*e.g.,* radio-labels, fluorescently labels, particulate labels.). For example, a CA-III probe may be immobilised *i.e.* cannot be routinely washed off) not wash onto a nitrocellulose membrane and exposed to the sample. Bound CA-III can be detected using a particle-labeled antibody, or primary and optionally secondary antibody immunoassays to arrive at a concentration of AC-III present in the sample. The roles of a CA-III and CA-III probe may be switched; the skilled person may adapt the method so CA-III probe is applied to immobilised CA-III to determine binding.

By solid support herein is meant any solid support which is capable of immobilising components and/or samples. Such solid supports are known in the art and include, but are not limited to, nitrocellulose, glass slides, nylon, silane coated slides, nitrocellulose coated slides, plastics, ELISA trays, magnetic beads. A solid support may be capable of holding single spotted sample, multi-samples and/or micro-arrays etc.

Where an immunoassay is employed, such immunoassay methods include, but are not limited to, dot blotting, western blotting, competitive and noncompetitive protein binding assays, enzyme-linked immunosorbant assays (ELISA), immunohistochemistry, fluorescence activated cell sorting (FACS), and others commonly used and widely described in scientific, patent literature, or employed commercially.

Biochemical assays such as the ELISA may rely on a colour change to indicate the presence or absence of CA-III in a sample, and to provide an indication of concentration by virtue of intensity. Since the colour change can be read by eye, such assays can be performed without resorting to instrumentation to read the result. Biochemical assays, therefore, may be employed outside the laboratory, for example, in the doctors surgery, by a visiting healthcare worker or as a home testing kit.

### Test strip and cartridge

Another way to perform a biochemical assay is to use a test-strip and labelled antibodies which combination does require any washing of the membrane. The test strip is well known, for example, in the field of pregnancy testing kits where an anti-hCG antibody is present on the support, and is carried complexed with hCG by the flow of urine onto an immobilised second antibody that permits visualisation.

In one preferred embodiment of the invention, is a solid support having a proximal and distal end, comprising:
- a sample application zone in the vicinity of the proximal end,
- a reaction zone distal to the sample application zone, and
- a detection zone distal to the reaction zone,
whereby said support has a capillary property that directs a flow of fluid sample applied in the application zone in a direction from the proximal end to the distal end.

The reaction zone comprises one or more bands of CA-III probe conjugated to a detection agent (e.g. colloidal gold) which CA-III probe conjugate is disposed on the solid support such that it can migrate with the capillary flow of fluid *i.e.* it is not immobilised. The detection zone comprises one or more capture bands comprising a population of CA-III probe immobilised on the solid support.

When a sample is applied to the sample application zone, it migrates towards the reaction zone by capillary flow. Any CA-III present in the sample reacts with the CA-III probe conjugate, and the complex so formed is carried by capillary flow to the detection zone. The detection zone, having CA-III probe permanently immobilised thereon, captures and immobilises any complex, resulting in a localised concentration of conjugate that can be visualised.

The zones as described herein generally do not overlap. They may be adjacently arranged with an absence or presence of an intervening gap of solid support devoid of band. A band may be disposed on a solid support by any means, for example, absorbed, adsorbed, coated, covalently attached or dried, depending on whether the reagent is required to be mobilised or not.

Reference is made in the description below to the drawings which exemplify particular embodiments of the invention; they are not at all intended to be limiting. The skilled person may adapt the device and substituent components and features according to the common practices of the person skilled in the art.

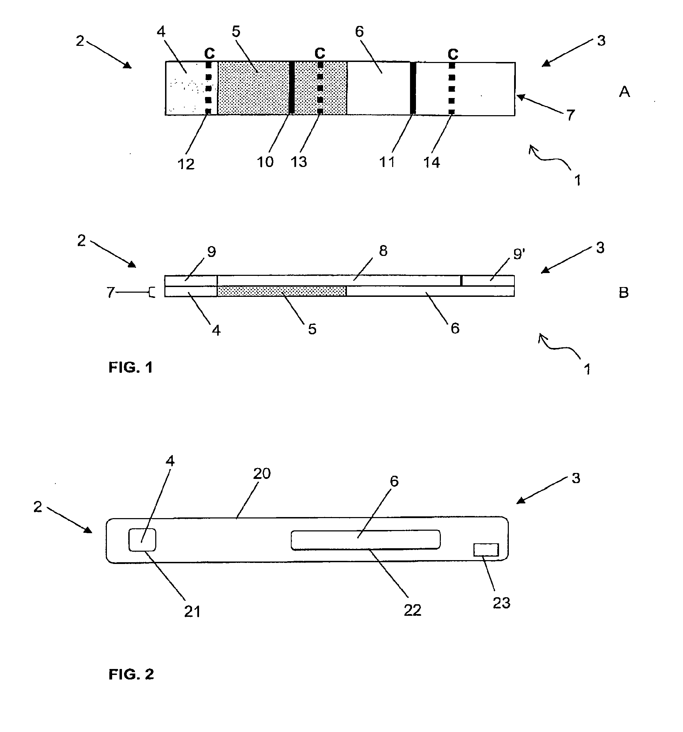

**FIG. 1A** and **B** shows a preferred embodiment of a test strip of the invention. The strip **1** includes a proximal end **2** and a distal end **3.** A sample application zone **4** is provided in the proximal end **2,** a reaction zone **5** is adjacent thereto and a detection zone **6** is in the vicinity of the distal end **3.**

A sample may be deposited onto the solid support **7** at the application zone **4** to transfer by capillary action to the detection zone **6.** A protective layer **8** that covers either or both the surfaces of the solid support **7,** except for a region of the sample application zone **4** may be provided. Such protective layer protects the sample and chemical constituency of the strip from contamination and evaporation.

One or more absorbent pads **9** in capillary contact with the sample application zone **4** of the solid support **7** may absorb and release sample as necessary; such pad **9** is typically placed on the surface of the solid support **7** that is the same or opposing the sample application zone **4.** In **FIG. 1** **B,** the absorbent pad **9** is part of the sample application zone **4.**

One or more other absorbent pads **9'** in capillary may be placed in contact with the detection zone **6** of the solid support **7,** distal to any capture bands **11, 14.** These pads **9'** may absorb fluid that has passed through the solid support; such pad **9'** is typically placed on the surface of the solid support **7** that is the same or opposing the sample application zone **4.**

The solid support **7** may made from any suitable material that has a capillary action property, and may have the same properties as described above. It should also be capable of supporting a substance (e.g. non-immobilised CA-III probe), which, when hydrated, can migrate across the solid support by a capillary action fluid flow.

The solid support **7** may also comprise a band of CA-III probe conjugate **10,** located in the reaction zone **5,** at a position distal to the sample application zone **4.** Any CA-III in the sample is carried by capillary action towards this band **10,** where it reacts with the permanently immobilised CA-III probe conjugate.

The CA-III probe conjugate may be associated with or attached to a detection agent to facilitate detection. Examples of lab detection agents include, but are not limited to, luminescent labels; colorimetric labels, such as dyes; fluorescent labels; or chemical labels, such as electroactive agents (e.g., ferrocyanide); enzymes; radioactive labels; or radiofrequency labels.

More commonly, the detection agent is a particle. Examples of particles useful in the practice of the invention include, but are not limited to, colloidal gold particles; colloidal sulphur particles; colloidal selenium particles; colloidal barium sulfate particles; colloidal iron sulfate particles; metal iodate particles; silver halide particles; silica particles; colloidal metal (hydrous) oxide particles; colloidal metal sulfide particles; colloidal lead selenide particles; colloidal cadmium selenide particles; colloidal metal phosphate particles; colloidal metal ferrite particles; any of the above-mentioned colloidal particles coated with organic or inorganic layers; protein or peptide molecules; liposomes; or organic polymer latex particles, such as polystyrene latex beads. Preferable particles are colloidal gold particles. The size of the particles may be related to porosity of the membrane strip: the particles are preferably sufficiently small to be transported along the membrane by capillary action of fluid.

Colloidal gold may be made by any conventional means, such as the methods outlined in G. Frens, 1973 Nature Physical Science, 241:20 (1973). Alternative methods may be described in U.S. Pat. Nos. 5,578,577, 5,141,850; 4,775,636; 4,853,335; 4,859,612; 5,079,172; 5,202,267; 5,514,602; 5,616,467; 5,681,775.

The selection of particle size may influence such factors as stability of bulk sol reagent and its conjugates, efficiency and completeness of release of particles from the test strip, speed and completeness of the reaction. Also, particle surface area may influence stearic hindrance between bound moieties.

The number of particles present in the CA-III probe conjugate may vary, depending on the size and composition of the particles, the composition of the solid support, and the level of sensitivity of the assay.

The solid support **7** further comprises one or more capture bands **11** in the detection zone **10.** A capture band comprises a population of CA-III probe permanently immobilised thereon. The CA-III:CA-III probe conjugate complex formed in the reaction zone **5** migrates towards the detection zone **6** where said band **11** captures migrating complex, and concentrates it, allowing it to be visualised either by eye, or using a machine reader. The CA-III probe present in the reaction zone **5** and in the detection zone **6** may reaction to the same part of CA-III or may react to different parts of CA-III.

One or more controls bands **12** may be present on the solid support **7.** For example, a non-immobilised peptide **12** might be present in the sample application zone **4,** which peptide does not cross-react with any of bands of CA-III probes **13, 14.** As the sample is applied, it migrates towards the reaction zone **5,** where an anti-peptide antibody conjugate is disposed **13,** and where a complex peptide-antibody complex is formed. Said complex migrates towards the detection zone **6,** where a capture band **14** of anti-peptide antibody is immobilised on the solid support, and which concentrates said complex enableing visualisation. The control capture band **14** is located separately from the CA-III capture band **11,** therefore, a positive reaction can be seen distinct from the detection reaction if the assay is working correctly.

A particular advantage of a control according to the invention is that they are internal controls - that is, the control against which the CA-III measurement results may be compared is present on the individual solid support. Therefore, the controls according to the invention may be used to correct for variability in the solid support, for example. Such correction would be impractical with external controls that are based, for example, on a statistical sampling of supports. Additionally, lot-to-lot, and run-to-run, variations between different supports may be minimized by use of control binding agents and control agents according to the invention. Furthermore, the effects of non-specific binding may be reduced. All of these corrections would be difficult to accomplish using external, off-support, controls.

During the assay, CA-III from the sample and the CA-III probe conjugate combine and concentrate on the solid support **7.** This combination results in a concentration of compounds that may can be visualised above the background colour of the solid support **7.** The compounds may be formed from a combination of above-mentioned compounds, including antibodies, detection agents, and other particles associated with the reaction and detection zones. Based on the particular assay being performed, the reaction and detection zones may be selectively implemented to achieve an appropriate dynamic range which may be linear or non-linear.

A solid support **7** for performing the assay may be housed within the cartridge **20** as shown, for example, in **FIG. 2****.** The cartridge is preferably watertight against urine, except for one or more openings. The solid support **7** may be exposed through an opening **21** in the cartridge to provide an application zone **4** in proximal end **2,** and another opening **22** to enable reading of detection zone **6** close to the distal end **3.** Cartridge **20** may include a sensor code **23** for communicating with a reading device.

### Surface plasmon resonance

The presence and/or concentration of CA-III in a sample can be measured by surface plasmon resonance (SPR) using a chip having CA-III probe immobilized thereon. Surface plasmon resonance monitors the change in mass near the immobilised sensor. This change in mass is measured as resonance units versus time after injection or removal of the sample, and is measured using a Biacore Biosensor (Biacore AB). CA-III probe can be immobilised on a sensor chip (for example, research grade CM5 chip; Biacore AB) according to methods described by Salamon *et al.* (Salamon et al., 1996, Biophys J. 71: 283-294; Salamon et al., 2001, Biophys. J. 80: 1557-1567; Salamon et al., 1999, Trends Biochem. Sci. 24: 213-219, each of which is incorporated herein by reference.). Conditions for CA-III binding to CA-III probe in an SPR assay can be fine-tuned by one of skill in the art using the conditions reported by Sarrio *et al.* (Sarrio et al., 2000, Mol. Cell. Biol. 20: 5164-5174, incorporated herein by reference) as a starting point. Binding reactions can be performed at different concentrations of immobilized CA-III probe, if necessary, to arrive at a concentration of CA-III in the sample. If a qualitative result is desired, controls and different concentrations may not be necessary. While CA-III probe is immobilised in the above, the skilled person may readily adapt the method so that the sample is the immobilised component.

### Fluorescence resonance energy transfer

Another method of determining the concentration of CA-III in a sample is by using fluorescence resonance energy transfer (FRET). FRET is a quantum mechanical phenomenon that occurs between a fluorescence donor (D) and a fluorescence acceptor (A) in close proximity to each other (usually < 100 angstroms of separation) if the emission spectrum of D overlaps with the excitation spectrum of A. The molecules to be tested, e.g., CA-III and CA-III probe, are labelled with a complementary pair of donor and acceptor fluorophores. While bound closely together by the CA-III: CA-III probe interaction, the fluorescence emitted upon excitation of the donor fluorophore will have a different wavelength than that emitted in response to that excitation wavelength when the CA-III and CA-III probe are not bound, providing for quantitation of bound versus unbound molecules by measurement of emission intensity at each wavelength. Donor fluorophores with which to label the sclerostin are well known in the art. Of particular interest are variants of the A. victoria GFP known as Cyan FP (CFP, Donor (D)) and Yellow FP (YFP, Acceptor(A)). As an example, the YFP variant can be made as a fusion protein with sclerostin. Vectors for the expression of GFP variants as fusions (Clontech) as well as fluorophore-labelled hCG mimetic compounds (Molecular Probes) are known in the art. Binding reactions can be performed at different CA-III probe concentrations, if necessary, to arrive at a concentration of CA-III. If a qualitative result is desired, controls and different concentrations may not be necessary.

### Bioluminescence resonance energy transfer

Another detection system is bioluminescence resonance energy transfer (BRET), which uses light transfer between fusion proteins containing a bioluminescent luciferase and a fluorescent acceptor. In general, one molecule of the CA-III:CA-III probe complex is fused to a luciferase *(e.g. Renilla* luciferase (Rluc)) - a donor which emits light in the wavelength of ~395 nm in the presence of luciferase substrate (e.g. DeepBlueC). The other molecule of the pair is fused to an acceptor fluorescent protein that can absorb light from the donor, and emit light at a different wavelength. An example of a fluorescent protein is GFP (green fluorescent protein) which emits light at ~510 nm. The addition of acceptor-fused candidate CA-III to the donor fused-CA-III probe will result in an energy transfer evidenced by, for example, an increase in acceptor fluorescence relative to a sample where an acceptor-fused CA-III does not bind. By measuring the interaction under a range of concentrations and conditions, if necessary, will provide the concentration of CA-III in a sample. If a qualitative result is desired, controls and different concentrations may not be necessary.

### Quenching

A variation on FRET uses fluorescence quenching to monitor molecular interactions. One molecule in the interacting pair can be labelled with a fluorophore, and the other with a molecule that quenches the fluorescence of the fluorophore when brought into close apposition with it. A change in fluorescence upon excitation is indicative of a change in the association of the molecules tagged with the fluorophore:quencher pair. Generally, an decrease in fluorescence of the labelled sclerostin is indicative that a candidate minetic bearing the quencher has been bound. Of course, a similar effect would arise when mimetic is fluorescently labelled and sclerostin bears the quencher. Binding reactions can be performed at different mimetic and/or sclerostin concentrations if necessary to arrive at a binding constant. For quenching assays, a 10% or greater (e.g., equal to or more than 20%, 30%, 40%, 50%, 60%) decrease in the intensity of fluorescent emission, indicates that the candidate hCG mimetic binds sclerostin. Control experiments using quench-labelled sclerostin and hCG can establish expected levels of quenching; a quenching observed with an hCG mimetic would be at least 10% (e.g., at least 20%, 30%, 40%, 50%, 60%, 70%, 80% or 90%) of the level observed with hCG.

In addition to the surface plasmon resonance and FRET methods, fluorescence polarization measurement is useful to quantitate binding. The fluorescence polarization value for a fluorescently-tagged molecule depends on the rotational correlation time or tumbling rate. Complexes, such as those formed by CA-III associating with a labeled CA-III probe, have higher polarization values than uncomplexed, labeled CA-III probe. Binding reactions can be performed at different CA-III probe concentrations if necessary to arrive at a concentration for CA-III in the sample. Control experiments using CA-III and CA-III probe can establish expected levels of polarization. If a qualitative result is desired, controls and different concentrations may not be necessary.

Any of the binding assays described can be used to determine the presence and/or concentration of CA-III in a urine sample. To do so, CA-III-probe is reacted with a sample, and the concentration of CA-III is measured as appropriate for the binding assay being used.

To validate and calibrate an assay, control reactions using different concentrations of standard CA-III and/or CA-III probe can be performed. Where solid phase assays are employed, after incubation, a washing step is performed to remove unbound CA-III. Bound, CA-III is measured as appropriate for the given label (e.g., scintillation counting, fluorescence, antibody-dye etc.). If a qualitative result is desired, controls and different concentrations may not be necessary. Of course, the roles of CA-III and CA-III probe may be switched; the skilled person may adapt the method so CA-III probe is applied to sample, at various concentrations of sample.

### CA-III probe

A CA-III probe according to the invention is any substance that binds specifically to CA-III. Examples of a CA-III probe useful according to the present invention, includes, but is not limited to an antibody, a polypeptide, a peptide, a lipid, a carbohydrate, a nucleic acid, peptide-nucleic acid, small molecule, small organic molecule, or other drug candidate. A CA-III probe can be natural or synthetic compound, including, for example, synthetic small molecule, compound contained in extracts of animal, plant, bacterial or fungal cells, as well as conditioned medium from such cells. Alternatively, CA-III probe can be an engineered protein having binding sites for CA-III. According to an aspect of the invention, a CA-III probe binds specifically to CA-III with an affinity better than 10⁻⁶ M.

A suitable CA-III probe can be determined from its binding with a standard sample of CA-III. Methods for determining the binding between CA-III probe and CA-III are described above.

A CA-III probe useful according to the present invention may be an antibody or antigen-binding fragment thereof which specifically binds to CA-III. As used herein, the term antibody includes, but is not limited to, polyclonal antibodies, monoclonal antibodies, humanised or chimeric antibodies, engineered antibodies, and biologically functional antibody fragments (e.g. scFv, nanobodies, Fv, etc) sufficient for binding of the antibody fragment to the protein.

Such antibody may be commercially available antibody against CA-III, such as, for example, mouse monoclonal antibody clone 2CA-4 (Spectral).

According to one aspect of the invention, the CA-III probe is labelled with a tag that permits detection with another agent (e.g. with a probe binding partner). Such tags can be, for example, biotin, streptavidin, his-tag, myc tag, maltose, maltose binding protein or any other kind of tag known in the art that has a binding partner. Example of associations which can be utilised in the probe:binding partner arrangement may be any, and includes, for example biotin:streptavidin, his-tag:metal ion (e.g. Ni²⁺), maltose:maltose binding protein.

### Diagnostic kit

One embodiment of the present invention is a kit for diagnosing a condition related to dysfunction of the PRT in a subject. Another embodiment of the present invention is a kit for monitoring the progress of a condition related to dysfunction of the PRT in a subject. Another embodiment of the present invention is a kit for monitoring the effectiveness of treatment of a subject with an agent. Said kits may comprise one or more of the following components:
- CA-III probe, preferably antibody directed against CA-III
- solid support provided with or coated with CA-III probe,
- control standards comprising CA-III,
- a solid support having fluid capillary properties comprising:
   - a distal and proximal end,
   - a sample application zone in the vicinity of the proximal end,
   - a reaction zone distal to the sample application zone,
   - a detection zone distal to the reaction zone,
   - where the reaction zone is disposed with CA-III probe labelled with detection agent, that can migrate towards the distal end in a flow of capillary action,
   - where the detection zone comprises immobilised CA-III probe that can capture CA-III.
- solid support having fluid capillary properties, housed in a cartridge as described herein.
- Urine sample container

### EXAMPLES

In this study, we have used Clcn5 knockout mice, as a well-defined model of renal Fanconi syndrome, in order to investigate the metabolic consequences and adaptative mechanisms to PT dysfunction. The *Glcn5*^{*Y*/*-*} kidneys were characterized by higher cell proliferation and oxidative stress, and a 4-fold upregulation of CAIII - which was previously unknown in the kidney. CAIII was exclusively distributed in the cytosol of scattered PT cells. These findings were confirmed in kidney samples from a patient with Dent's disease, and extended to other mouse models of PT dysfunction and PT cell lines exposed to oxidant conditions. As a whole, these results show that generalized PT dysfunction is associated with increased cell proliferation, dedifferentiation and oxidative stress. The kidney-specific upregulation of CAIII, which is an early mesodermal marker, may play a role in PT cell defence against oxidative damage.

### 1. Materials and Methods

### Mouse models.

Experiments were conducted on 12-week-old and 1-year-old *Glcn5* wild-type (Y/+) and KO (Y/-) mice. The *Clcn5*^{*Y*/*-*} mice, generated by targeted deletion of the exon VI of *Clcn5,* have been extensively characterized and mimic the phenotype of human Dent's disease (Wang, S. S. et al. 2000 Hum. Mol. Genet. 9: 2937-2945.). Animals were kept in metabolic cages for 24h with *ad libitum* access to food and drinking water, and urine was collected on ice-cold Complete^{™} protease inhibitors (Roche, Vilvoorde, Belgium). Kidneys from two additional mouse models of human renal Fanconi syndrome of variable severity, *i*.*e*. 15-week-old *megalin* KO mice (Willnow, T. E. et aL 1996 Proc. Natl. Acad. Sci. U. S. A. 93: 8460-8464.), and 24-week-old *Ctns* KO mice (Cherqui, S. et al. 2002, Mol. Cell Biol. 22: 7622-7632.) were also used. The *megalin* KO mice exhibit a specific defect in PT endocytic apparatus resulting in impaired uptake of filtered LMW proteins, without significant alteration of glucose, electrolyte and amino acid transports (Leheste, J. R et al. 1999. Am. J. Pathol. 155: 1361-1370.). The *Ctns* KO mice present no signs of proximal tubulopathy despite the severe PT defects observed in children with infantile cystinosis, which suggests alternative rescue pathways in mouse (Cherqui, S. et al., 2002, Mol. Cell Biol. 22: 7622-7632.). All samples were obtained in accordance with NIH guidelines for the care and use of laboratory animals, and with the approval of the Committee for Animal Rights of the UCL Medical School.

### Human samples.

Frozen and formalin-fixed kidney samples were obtained at time of bilateral nephrectomy in an end-stage patient with Dent's disease. The clinical features of this patient, who harbours the missense mutation Gly506GIu in *CLCN5,* were reported previously (Lloyd, S.E. et al. 1996. Nature. 379: 445-449.). Four end-stage kidneys (chronic interstitial nephritis) removed during renal transplantation were used as controls. These samples were snap-frozen in liquid nitrogen and stored at -80°C, or routinely fixed in 4% formaldehyde. The use of these samples has been approved by the Ethical Review Board of the UCL Medical School.

### Proximal tubule cell lines.

The human kidney (HK2) and *Opossum* kidney (OK) cell lines are established models of PT cells (Ryan, M. J. et al. Kidney Int. 45: 48-57.). The HK-2 cell line was obtained from ATCC (Teddington, UK) and grown on keratinocyte-serum free medium (GIBCO-BRL 17005-042, Invitrogen) with 5 ng/ml recombinant epidermal growth factor, 50 µg/ml bovine pituitary extract, 50 U/ml penicillin, and 50 µg/ml streptomycin, at 37° C in a 95% air / 5% CO₂ incubator. The OK cells were grown on DMEM-F12 medium (GIBCO-BRL 31330-038, Invitrogen), with 10 U/ml penicillin, 10 µg/ml streptomycin, and foetal bovine serum 10%, at 37° C in a 95% air / 5% CO₂ incubator. When the cultures reached confluence, subcultures were prepared using a 0.02% EDTA - 0.05% trypsin solution (Invitrogen). After 24h-deprivation of serum, HK-2 cells (passage 12) and OK cells (passage 111) were treated with H₂O₂ (1 mM or 0.3 mM, respectively). At various times post H₂O₂-treatment, cells were trypsinized, washed twice in cold PBS, and centrifuged at 300 *g* for 5 min. The pellet was harvested at -80°C before mRNA and protein extraction.

### RNA extraction and double strand cDNA synthesis.

Total RNA was extracted from frozen samples (human and mouse kidneys, cell lysates) using Trizol reagent (Invitrogen, Merelbeke, Belgium). The concentration of each RNA sample was obtained from optical densitometry (260 nm) measurements and RNA quality was confirmed using agarose gel electrophoresis. For AFLP, poly(A)+ RNA were prepared from 75 *µ*g of total RNA using Dynabeads Oligo(dT)₂₅ (Invitrogen). First strand cDNA was synthesized from 500 ng of Poly(A)+ RNA using Superscript It RNase H- Reverse Transcriptase (Invitrogen) in a total volume of 20 µl at 37°C for 50 min. Double strand cDNA was synthesised in the same vial using T4 DNA Polymerase and purified using QlAquick Extraction Kit (Qiagen, Venlo, The Netherlands).

### AFLP reactions.

The AFLP protocol was performed as previously described (42). cDNA samples were digested with *Eco*RI and *Msel* (Fermentas, Vilnius, Lithuania) for 2 h at 37°C. Restriction fragments were next ligated to *Eco*RI and Msel double strand adapters (Table 2) for 2 h at 20°C.

**Table 2. Nucleotide sequence of adapters and primers used for AFLP reactions**

| **Name** | **Sequence** |
|---|---|
| Ad1-*Eco* | 5' CTCGTAGACTGCGTACC 3' |
| Ad2-*Eco* | 5' AATTGGTACGCAGTCTAC 3' |
| Ad1-*Mse* | 5' GACGATGAGTCCTGAG 3' |
| Ad2-Mse | 5' TACTCAGGACTCAT 3' |
| *Eco*-P0 | 5' GACTGCGTACCAATTC 3' |
| *Mse*-P0 | 5' GATGAGTCCTGAGTAA 3' |
| *Eco*-PAA | 5' GACTGCGTACCAATTCAA 3' |
| Eco-PAC | 5' GACTGCGTACCAATTCAC 3' |
| *Eco*-PAG | 5' GACTGCGTACCAATTCAG 3' |
| Eco-PAT | 5' GACTGCGTACCAATTCAT 3' |
| *Mse-PAA* | 5' GATGAGTCCTGAGTAAAA 3' |
| Mse-PAC | 5' GATGAGTCCTGAGTAAAC 3' |
| *Mse*-PAT | 5' GATGAGTCCTGAGTAAAT 3' |

The restriction fragments with ligated adapters were diluted (10X) with TE buffer (100 mM Tris-HCI, 10 mM EDTA, pH 8.0), and used as a template for the pre-amplification reaction which was performed for 20 cycles (94°C, 30 sec; 56°C, 1 min; 72°C, 1 min) using *Eco-*P0 and *Mse*-P0 primers. The product was diluted (10X) with TE buffer and 5 µl were used for selective amplification, as follows: 33 cycles including 9 touchdown cycles comprising a decrease of the annealing temperature from 65°C to 56°C, which was maintained for 24 cycles. Twelve primer combinations were used for selective amplification: Eco-PAA and Mse-PAA or Mse-PAC or Mse-PAT, Eco-PAC and Mse-PAA or Mse-PAC or Mse-PAT, Eco-PAG and Mse-PAA or Mse-PAC or Mse-PAT, Eco-PAT and Mse-PAA or Mse-PAC or Mse-PAT. All amplification reactions were performed in the iCycler thermal cycler (Bio-Rad, Nazareth, Belgium). Selective amplification products were denatured at 95°C for 3 min and separated on sequencing gels (6% polyacrylamide, 6 M urea) that were then dried and exposed to Kodak BioMax film (Amersham Biosciences, Buckinghamshire, UK) overnight at -80°C. The bands of interest were removed from the gel and soaked in water. AFLP fragments were recovered by PCR under the same conditions as used for the selective amplification. Reamplified cDNAs were visualised on a 1.5% (w/v) agarose gel, subcloned into pGEM-T easy vector (Promega, Leiden, The Netherlands) and sequenced (Genome Express, Meylan, France). Each reamplified AFLP fragment was compared against all sequences in the non-redundant databases using BLAST sequence alignment program: http://www.ncbi.nlm.nih.qov/BLAST/ (Altschul, S. F. et al. J. Mol. Biol. 215: 403-410).

### Real-time RT-PCR.

Total RNA was treated with DNase I (Invitrogen) and reverse-transcribed into cDNA using SuperScript III RNase H- Reverse Transcriptase (Invitrogen). Changes in mRNA expression levels were determined by real-time RT-PCR (iCycler IQ System, Bio-Rad) using SYBR Green I (Invitrogen) detection of single PCR product accumulation. Specific primers were designed using Beacon Primer Designer 2.0 (Premier Biosoft International, Palo Alto, CA) and are listed in Table 3.

**Table 3 Primers used for real-time RT-PCR**

| **Primer pair** | **Forward** | | **Length** | **Efficiency** |
|---|---|---|---|---|
| | | **Reverse** | **(bp)** | |
| **Mouse** | | | | |
| | 5'CTTGAAGCACTGCATTCCAT 3' | 5'CACGATCCAGGTCACA CATT 3' | 153 | 1.03±0.09 |

| **car2** | | | | |
|---|---|---|---|---|
| | 5'CTTGATGC CCTGGACAAAAT 3' | 5'GAGCCGTGGTAGGTCCAATA 3' | 110 | 1.04±0.11 |

| **car3** | | | | |
|---|---|---|---|---|
| *PCNA* | 5'TTGGAATCCCAGAACAGGAG 3' | 5'ATTGCCAAGCTCTCCACTTG 3' | 155 | 1.02±0.20 |
| | 5'TGCAAAGGTAGAGGCTCCAT 3' | 5'CAGGTAGGCCAGAGCAAGT 3' | 152 | 1.03±0.17 |

| **K167** | | | | |
|---|---|---|---|---|
| *osteopontin* | 5'TCCAATCGTCCCTACAGTCG 3' | 5'CGCTCTTCATGTGAGAGGTG 3' | 146 | 0.98±0.08 |
| *catalase* | 5'CATGGTCTGGGACTTCTGGA 3' | 5'GACTGCCTCTCCATCTGCAT 3' | 151 | 0.97±0.27 |
| *Type* / *SOD* | 5' GGGTTCCACGTCCATCAGTA 3' | 5'CAGTCACATTGCCCAGGTCT 3' | 136 | 1.10±0.09 |
| *thioredoxin* | 5'TGATCAAGCCCTTCTTCCAT 3' | 5'CCCACCTTTTGACCCTTTTT 3' | 151 | 1.00±0.20 |
| | 5'TGCACCACCAACTGCTTAGC 3' | 5' GGATGCAGGGATGATGTTCT 3' | 176 | 1.04±0.03 |

| **gapdh** | | | | |
|---|---|---|---|---|
| | | | | |
| *hprt1* | 5'ACATTGTGGCCCTCTGTGTG 3' | 5'TTATGTCCCCCGTTGACTGA 3' | 162 | 0.99±0.01 |

| ***Human*** | | | | |
|---|---|---|---|---|
| | 5' CCCTGGATGGCACTTACAG 3' | 5'CAGCTTTCCCAAAATCCCCA CATT 3' | 3' 153 | 1.01 ± 0.10 |

| **car2** | | | | |
|---|---|---|---|---|
| | 5'GCCGGGACTACTGGACCTA 3' | 5'CGTTCTCAGCACTGGAGAG 3' | 144 | 0.97 ± 0.11 |

| **car3** | | | | |
|---|---|---|---|---|
| | 5' ACGTCTCTTTGGTGCAGCTC 3' | 5' GCGTTATCTTCGGCCCTTAG 3' | 157 | 0.98 ± 0.30 |

| **PCNA** | | | | |
|---|---|---|---|---|
| *osteopontin* | 5' ATGGCCGAGGTGATAGTGTG 3' | 5' GATGGCCTTGTATGCACCAT 3' | 146 | 1.10 ± 0.30 |
| *catalase* | 5' TGGCTCATTTTGACCGAGAG 3' | 5' GCGATGGGAGTCTTCTTTCC 3' | 148 | 0.95 ± 0.26 |
| *thioredoxin* | 5' TCAGCCACGTGGTGTGGG 3' | 5' TGGAATGTTGGCATGCATTTGA 3' | 152 | 1.20 ± 0.30 |
| *GAPDH* | 5' GGGGCTCTCCAGAACATCAT 3' | 5' TCTAGACGGCAGGTCAGGT 3' | 149 | 0.97 ± 0.12 |

The PCR products were size-fractionated on 1.5% agarose gel, stained with ethidium bromide, purified by QIAquick Gel Extraction Kit (Qiagen) and sequenced by Genome Express. Real-time RT-PCR analyses were performed in duplicate with 200 nM of both forward and reverse primers in a final volume of 25 µl using 1 Unit of Platinum Taq DNA Polymerase, 6 mM MgSO₄, 400 *µ*M dNTP and SYBR Green I diluted 1/100,000. The PCR mix contained 10 nM fluorescein for initial well-to-well fluorescence normalization. PCR conditions were as follows: 94°C for 3 min, 40 cycles of 30 sec at 95°C, 15 sec at 61 °C and 1 min at 72°C. The melting temperature of the PCR product was checked at the end of each PCR by recording SYBR Green fluorescence increase upon slow renaturing DNA. For each assay, standard curves were prepared by serial 4-fold dilutions of WT mouse kidney cDNA. The efficiencies of the amplifications with each primer set were calculated from the slope of the standard curve [efficiency = (10^{-1/slope}) - 1] and were close to 1 (Table 3). The relative changes in Target mRNA / GAPDH (or HPRT1) mRNA ratio between *Glcn5*^{*Y*/*+*} and *Clcn5*^{*Y*/*-*} samples were determined by using the formula: Efficiency ΔΔCt.

### Laser capture microdissection (LCM).

Frozen sections (7 *µ*m) of *Clcn5*^{*Y*/*+*} and *Clcn5*^{*Y*/*-*} kidneys were mounted on appropriate coated slides (PALM MembraneSlides, P.A.L.M. Microlaser Technologies AG, Bernried, Germany), washed in H₂O for 1 min, dehydrated in a stepwise manner with 70%, 95% and 100% ethanol for 30 sec each, and finally dried in xylene for 5 min. Samples of ~20,000 *µ*m² were selectively microdissected from cortex and medulla regions using PALM Microsystem (Leica, Wetzlar, Germany) following manufacturer's recommendations. The microdissected samples were transferred to an eppendorf coated with PCR oil (Sigma M5904), pooled (total area of ~140,000 *µ*m² from each region), incubated with 60 µl of RNA lysis buffer (Ambion, Huntington, The United Kindgdom), and further processed for RNA extraction and quantification.

### Antibodies.

Mouse monoclonal antibodies against CAIII (Spectral, Toronto, Canada) (Azzazy, H. M., P. J. Cummings, D. R. Ambrozak, R. H. Christenson. 1998. **17:** 553-558.); V-ATPase E1 subunit (a gift of Dr. S. Gluck, University of California, San Francisco, CA, USA); proliferative cell nuclear antigen (PCNA, clone PC-10, Dako, Heverlee, Belgium); and β-actin (Sigma, St-Louis, MO); rat monoclonal against Ki67 antigen (clone TEC-3, Dako); rabbit polyclonal antibodies against CAIII (Nishita, T., et al. Am. J. Vet. Res. 63: 229-235.) and aquaporin-1 (Chemicon, Temecula, CA); and sheep polyclonal antibodies against CAII (Serotec, Oxford, UK) were used.

### Protein extraction and immunoblotting.

Cytosolic proteins were extracted from kidney samples as previously described (Wang, S. S., O. Devuyst, P. J. Courtoy, X. T. Wang, H. Wang, Y. Wang, R. V. Thakker, S. Guggino, W. B. Guggino. 2000. Hum. Mol. Genet. 9: 2937-2945.). Briefly, tissues were homogenized in ice-cold buffer (0.25 M sucrose, 20 mM imidazole, pH 7.4, 1 mM EDTA) containing Complete^{™} protease inhibitors (Roche). A low-speed "nuclear" fraction was pelleted from the homogenate by centrifugation at 1000 *g* for 10 min and extracted twice by resuspension sedimentation. The resulting supernatant was centrifuged at 100,000 *g* for 60 min at 4°C in a 50Ti fixed-angle rotor (Beckman, Palo Alto, CA). The supernatant was considered as the cytosolic fraction, and the high-speed pellet as the membrane compartment. To obtain cell lysates, the HK-2 and OK cells were harvested by trypsinization, and centrifuged twice at 1000 x g for 10 min. After discarding the supernatant, the pellet was solubilized in ice-cold lysis buffer containing Complete MiniR (Roche), briefly sonicated (Branson Sonifier 250, 2 pulses at 40% intensity), and then centrifuged at 16,000 x g for one minute at 4°C. Protein concentrations were determined using bicinchoninic acid protein assay (Pierce, Aalst, Belgium). The proteins were separated through SDS-PAGE and transferred onto nitrocellulose membrane (Bio-Rad). After blocking, membranes were incubated overnight at 4°C with primary antibodies, rinsed and incubated for 1 h at room temperature with the appropriate secondary peroxidase-labelled antibody (Dako). The immunoreactive bands were detected using enhanced chemiluminescence (Amersham Biosciences). Normalization for β-actin was obtained after stripping the blots. Specificity of the immunoblot was determined by incubation with non-immune rabbit or mouse IgG (Vector Laboratories, Brussels, Belgium). Densitometry analysis was performed with a Canon CanoScan8000F using the NIH Image V1.60 software. All immunoblots were performed in duplicate.

### Immunostaining.

Kidney samples were fixed for 6 h at 4°C in 4% paraformaldehyde (Boehringer Ingelheim, Heidelberg, Germany) in 0.1 M phosphate buffer, pH 7.4, prior to embedding in paraffin. Six-µm thick sections were rehydrated and incubated for 30 min with 0.3% hydrogen peroxide to block endogenous peroxidases. After incubation with PBS 10% normal goat serum for 20 min, sections were incubated for 45 min with primary antibodies in PBS 2% BSA. After washes, sections were incubated with appropriate biotinylated secondary antibodies (Vector Laboratories), washed and incubated for 45 min with the avidin-biotin peroxydase complex (Vectastain Elite, Vector Laboratories) and aminoethylcarbazole. Control experiments included incubation (i) in the absence of primary antibody, (ii) with non-immune rabbit or mouse IgG (Vector Laboratories). Counting of PCNA-, Ki67-, and CAIII-positive PT cells was blindly performed on 5 different cortex areas in 4 pairs of *Clcn5*^{*Y*/*-*} vs. *Clcn5*^{*Y*/*+*} kidneys.

*Apoptosis assay.* Apoptotic cells were detected in kidneys using the terminal deoxynucleotidyl transferase-mediated deoxyuridine triphosphate nick end labeling (TUNEL) method (Cell Death detection kit, Roche). Sections were pre-treated with 20 µg/ml proteinase K for 20 min. Positive control sections were first treated with 100 µg/ml DNAse I for 10 min at room temperature, whereas omission of transferase was regarded as negative control.

### Detection of superoxide anion (O₂⁻) generation.

The *in situ* production of O₂⁻ in kidney sections was assessed using the hydroethidine (HE) fluorescence method (Piech, A., C. Dessy, X. Havaux, O. Feron, J.L. Balligand. 2003. Cardiovasc. Res. 57: 456-467). HE is freely permeable to cells, and is oxidized by O₂⁻ to red fluorescent ethidium bromide (EB). After excitation at 480 nm, EB emits light at a wavelength of 610 nm. After embedding in Tissue Tek OCT compound (Sakura Finetek, Zoeterwoude, The Netherlands), kidneys were snap-frozen in pre-cooled isopentane, cut into 5- µm-thick cryosections, and stored at -80°C. The 5mM stabilized HE solution in DMSO (Invitrogen) was diluted to 2 x 10⁻⁶ M in water before use. The tissue sections were incubated with 50 *µ*l of HE solution at 37°C for 30 min in a light-protected and humidified chamber. Red fluorescence from HE-treated samples was measured during 5 ms using the software KS400 (Zeiss, Zaventem, Belgium) through a Zeiss Axiovert S100 microscope equipped with an Axiocam camera.

### lmmunogold labelling.

Kidneys were fixed by retrograde perfusion through the aorta with 2% formaldehyde in 0.1 M sodium cacodylate buffer, pH 7.2. Tissues were trimmed into small blocks, further fixed by immersion for 1 h in 1% formaldehyde, infiltrated with 2.3 M sucrose for 30 min and frozen in liquid nitrogen. For electron microscopy (EM), 70-90 nm sections were obtained at -100°C with an FCS Reichert Ultracut S cryoultramicrotome as previously described (Christensen, E. I. 1995. Eur. J. Cell. Biol. 66: 349-364.). For EM immunolabeling the sections were incubated with rabbit anti-CAIII at 4°C overnight followed by incubation for 1 hour with 10 nM goat anti-rabbit gold particles (BioCell, Cardiff, UK). The cryosections were embedded in methylcellulose containing 0.3% uranyl acetate and studied in a Philips CM100 electron microscope. Control sections were incubated with secondary antibody alone or with non-specific rabbit serum.

### Statistics.

Results are expressed as means ± SD. Comparisons between groups were made by Student unpaired *t*-tests. The significance level was set at p<0.05.

### 2. Results

### 2.1. Metabolic outcomes of CIC-5 inactivation in mouse kidney

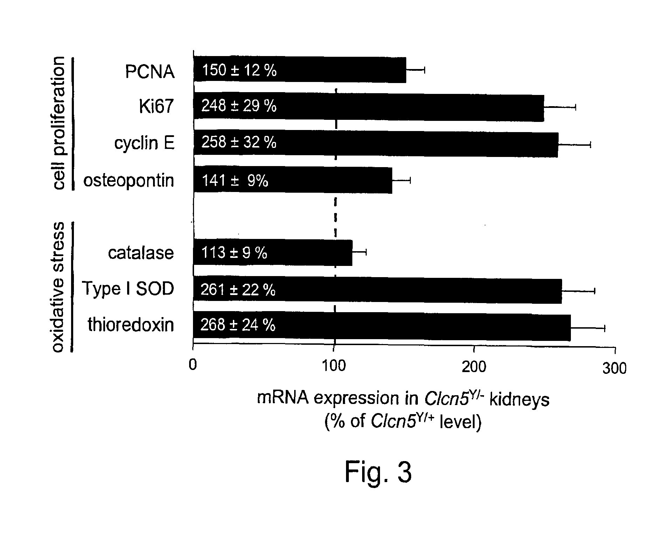

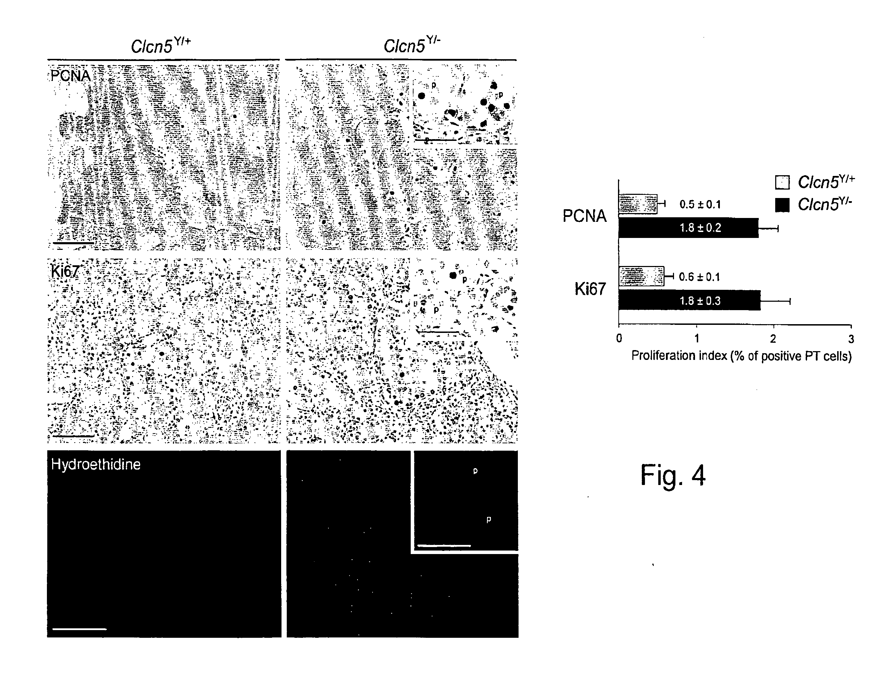

Real-time RT-PCR analyses showed a significant increase in differentiation markers, such as PCNA, Ki67, cyclin E and osteopontin, in the kidneys of *Clcn5*^{*Y*/*-*} mice, taken as a paradigm of renal Fanconi syndrome (**FIG. 3**). Furthermore, the increased mRNA expression of distinct reactive oxygen species (ROS) scavengers, such as type I superoxide dismutase (SOD) and thioredoxin, indicated an increased solicitation of cell oxidative defenses in *Clcn5*^{*Y*/*-*} kidneys in comparison to controls (**FIG. 3**). These results, obtained in 12-week-old mice, were also observed in 1-year-old *Clcn5^{Yl-}* mice (data not shown). Immunohistochemistry detected a significantly higher number of PCNA- and Ki67-positive cells in CIC-5 deficient kidneys (-1.8% of PT cells) vs. controls (-0.5% of PT cells) (**FIG. 4**). Comparative measurement of ethidium fluorescence in kidney sections revealed that the lack of CIC-5 was associated with a major increase in the production of superoxide O₂⁻ anion in PT cells (**FIG. 4**). These experiments showed that the PT dysfunction associated with inactivation of CIC-5 is reflected by a significant increase in cell proliferation and a major oxidative stress that solicitates ROS scavengers.

### 2.2. Comparison of Clcn5^{Y/}⁺ and Clcn5^{Y/}⁻ renal transcriptomes

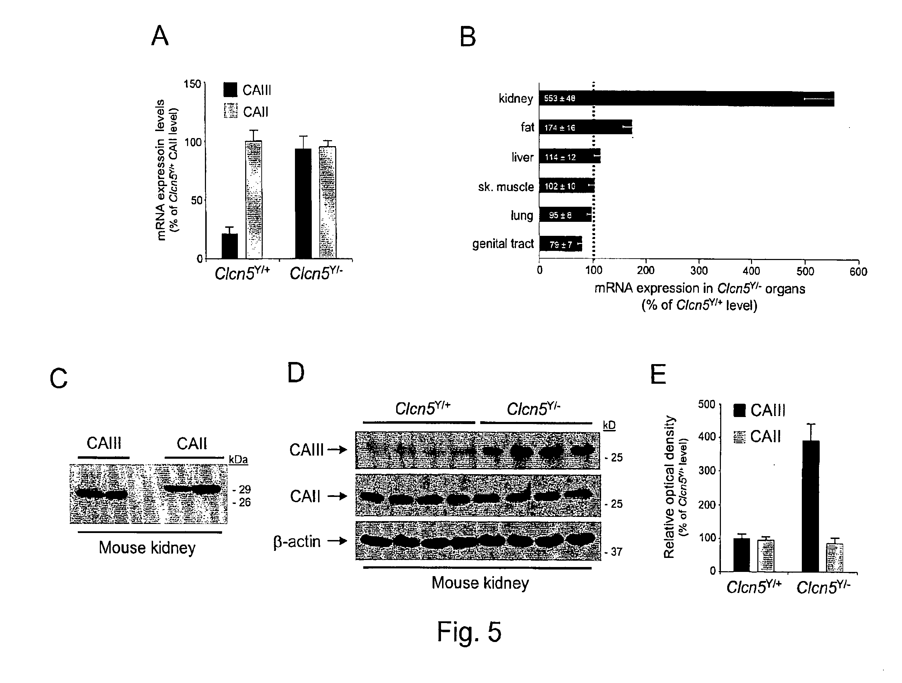

We next sought to identify differentially expressed genes possibly involved in adaptative mechanisms against PT dysfunction using the AFLP procedure on 12-week-old *Clcn5*^{*Y*/*-*} and *Clcn5*^{*Y*/*+*} kidneys (n = 4 pairs). Using one third of the possible AFLP primer combinations (see Materials and Methods), a total of 10 cDNA bands were reproducibly identified as differentially expressed in *Clcn5*^{*Y*/}*⁻ versus ClcnS*^{*Y*/*+*} kidneys. One of these bands, significantly upregulated in *Clcn5*^{*Y*/*-*} samples, was identified as a transcript of *Car3* (GenBank accession number: M27796), encoding Type III carbonic anhydrase (CAIII). The other cDNA bands, which were differentially expressed in *Clcn5*^{*Y*/}*⁻ vs.* control kidneys, corresponded to unidentified mouse contigs. Quantitative real-time RT-PCR was used to validate these results, by comparing the mRNA expression of CAIII and CAII, the most abundant CA isoform in the kidney, in *Clcn5*^{*Y*/}*⁻ vs. Clcn5*^{*Y*/*+*} kidneys (FIG. 5, panel A). As expected, CAIII mRNA expression was ~5-fold lower than CAII in wild-type kidneys. However, the CAIII transcript was significantly upregulated in CIC-5 deficient kidneys (Ratio: 553% ± 48 of *Clcn5*^{*Y*/*+*} level, n=6), whereas CAII mRNA expression remained unchanged (94% ± 8 of *Clcn5*^{*Y*/*+*} level). These results were confirmed using laser capture microdissected cortex samples (data not shown). A similar, 5-fold induction of CAIII expression was also observed in the kidneys from 1-year-old *Clcn5*^{Y/*-*} mice in comparison to age-matched controls (data not shown). The upregulation of CAIII mRNA associated with the loss of CIC-5 was specific to the kidney, as CAIII mRNA expression levels in liver, skeletal muscle *(vastus lateralis)* and lung from *Clcn5*^{*Y*/*-*} mice were unchanged (**FIG. 5**, panel B).

These data demonstrate the usefulness of the AFLP procedure coupled with real-time RT-PCR to compare the transcriptome of distinct groups of mice. Using this approach, we evidenced a significant and kidney-specific induction of *Car3* mRNA expression in mice lacking CIC-5.

### 2.3. Expression of CAIII in Clcn5^{Y/}⁺ and Clcn5^{Y/}⁻ kidney

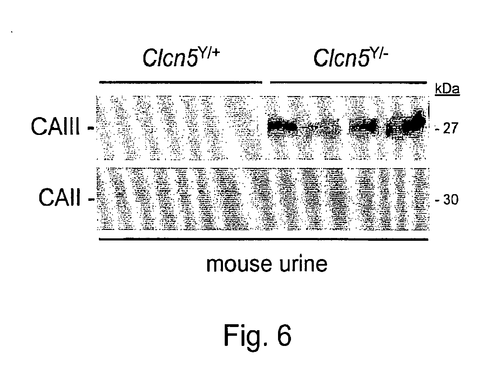

In order to investigate the upregulation of CAIII at protein level, we first validated the specificity of our antibodies raised against the closely related CAII and CAIII isoforms. There was no cross-reactivity between the two isozymes, characterized by a slightly distinct molecular weight after SDS-PAGE migration (**FIG. 5**, panel C). Using these antibodies, immunoblotting analyses confirmed the consistent, 4-fold upregulation of CAIII in 12-week-old CIC-5 deficient kidneys, contrasting with the lack of changes in CAII expression (panels D-E). These data were confirmed by using a commercial mouse monoclonal antibody directed against CAIII (data not shown). Of note, immunoblotting analyses also detected a specific excretion of CAIII in the urine of the *Clcn5*^{*Y*/*-*} whereas it was not detected in *Clcn5*^{*Y*/*+*} or any wild-type mouse tested (FIG. 6). These results support the data obtained by real-time RT-PCR, and further demonstrate that CAIII expression is significantly and specifically increased at both mRNA and protein levels in CIC-5 deficient kidneys. The detection of CAIII in *Clcn5*^{*Y*/*-*} urine also indicates that it represents a novel biomarker of PT dysfunction.

### 2.4. Cellular and subcellular distribution of CAIII in mouse kidney

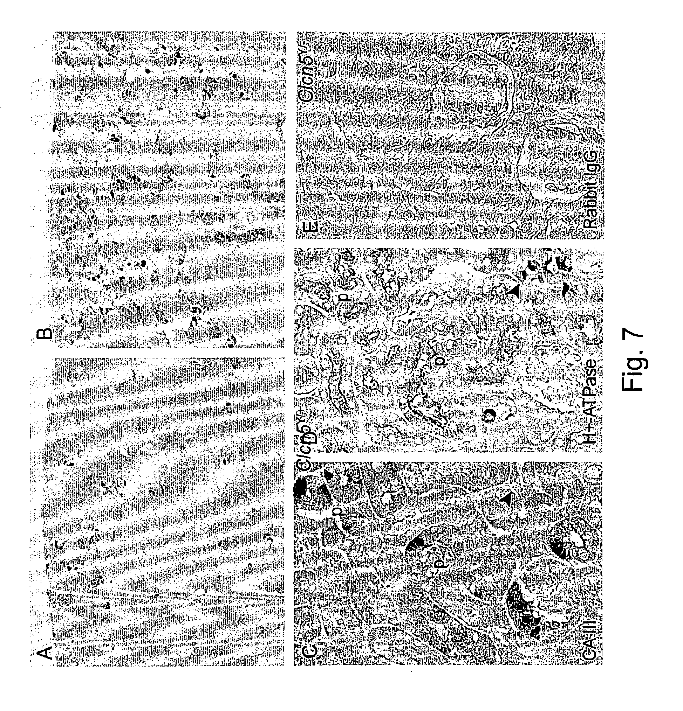

In normal kidney, a weak immunoreactive signal for CAIII was observed in some tubules located in the outer cortex (**FIG. 7**, panel A). At higher magnification, the staining pattern was restricted to a subset of cells lining the PT, identified by their apical reactivity for the E1 subunit of the V-ATPase (panels C-D). Of note, CAIII was detected only in PT cells, and was not observed in any other cell types including the α-type IC, which express the V-ATPase at their apical membrane (panels C-D, arrowheads). In CIC-5 deficient kidney, the total number of CAIII-positive PT cells in the outer cortex was increased -4-fold (17.1% vs. 4.2% of *Clcn5*^{*Y*/*+*} PT cells) (panel B). In addition, immunoreactive signal for CAIII was detected in PT of the inner cortex. No signal was detected with non-immune rabbit IgG (panel E). The number of PT cells undergoing apoptosis established by the TUNEL reaction was similar in *Clcn5*^{*Y*/*+*} and *Clcn5*^{*Y*/*-*} kidneys (data not shown).

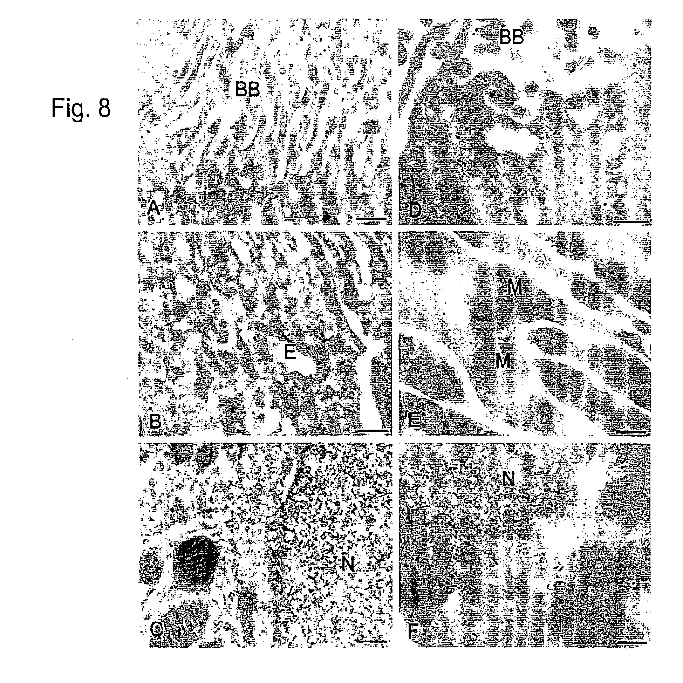

Subcellular fractionation of mouse kidneys demonstrated that CAIII was predominantly located in the cytosol, with residual distribution in membrane and nuclear fractions, as reported previously in adipocytes and hepatocytes (Tweedie, S., Y. Edwards. 1989. Biochem. Genet. 27: 17-30.). Immunogold analyses showed that in normal kidney cortex, CAIII distribution was mainly cytosolic, also including the apical brush border microvilli (**FIG. 8**, panels A and D). Nuclei were labelled (panels C and F), and a possible endosomal labelling could not be excluded (panel B). No significant signal was noticed in mitochondria (panel E). In *Clcn5^{Yl-}* samples, CAIII labelling appeared stronger than in *Clcn5*^{*Y*/*+*} kidneys, with a similar distribution (panels F vs. C). Altogether these data demonstrate that the CAIII isozyme is present in mouse kidney, with a segmental distribution including the cytosol, the nucleus and the brush border of a subset of cells lining the PT in the outer cortex. The loss of the Cl⁻ transporter CIC-5 causes a significant increase of CAI II expression in PT cells of both outer and inner cortices, with a similar subcellular localization.

### 2.5. Upregulation of CAIII in human Dent's disease kidney

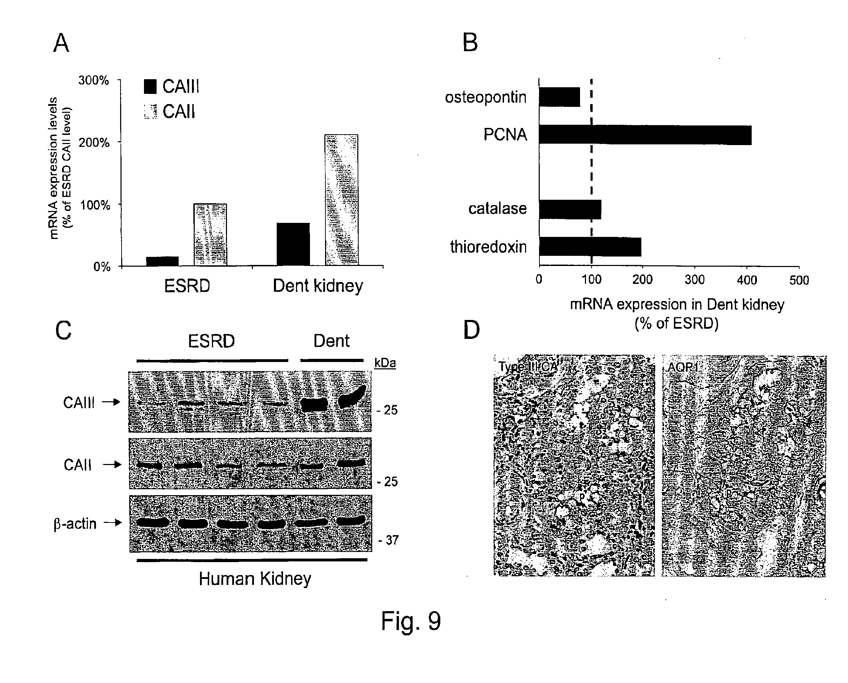

The data obtained in the *Clcn5*^{*Y*/*-*} mouse model were assessed in kidney samples from one patient with renal Fanconi syndrome due to the inactivating Gly506Glu mutation of *CLCN5* responsible for Dent's disease (Lloyd, S.E., 1996. *Nature.* **379**: 445-449.). There was a -5-fold upregulation of CAIII at both mRNA and protein levels in kidney samples with Dent's disease in comparison to 4 end-stage kidney samples taken as controls (**FIG. 9**, panels A and C). In addition, real-time RT-PCR studies showed that the functional loss of CIC-5 was associated with an induction of PCNA and thioredoxin expression, reflecting a higher cell proliferation and oxidative stress, respectively (panel B). Despite tissue damage due to the end-stage renal disease, the expression of CAIII could be located in PT cells, identified by co-staining with the water channel aquaporin-1 (panel D). These results demonstrate that the functional loss of CIC-5 in human kidney is associated with proliferation, protection against oxidative damage and induction of CAIII in PT cells.

### 2. 6. Expression of CAIII mRNA in distinct mouse models of PT dysfunction

As demonstrated above, the severe PT dysfunction caused by the functional loss of CIC-5 is associated with increased expression of CAIII in both mouse and human PT cells. In order to clarify whether CAIII induction was specifically caused by CIC-5 inactivation or participated in a common cellular response to PT dysfunction, CAIII mRNA expression was investigated in two additional mouse models of renal Fanconi syndrome, namely the megalin- and cystinosin-deficient mice. These models can be distinguished from each other by the severity of PT defects, as summarized in Materials and Methods. The expression of CAIII mRNA was significantly increased in *megalin* KO (262 ± 22% of WT level, n=3), whereas no changes were observed in *Ctns* KO samples. These results indicate that the induction of CAIII expression may correlate with the severity of PT dysfunction, suggesting that this isozyme participates in the general cellular response to PT dysfunction.

### 2.7. Induction of CAIII expression in PT cells exposed to H₂O₂

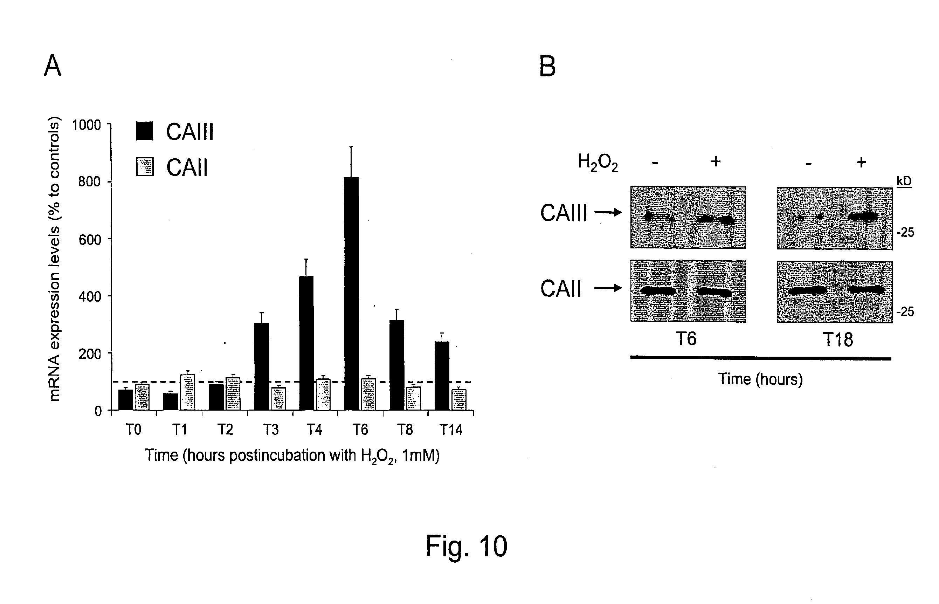

The HK-2 cell line is a well-established model for normal human PT cells (Ryan, M. J., G. Johnson, J. Kirk, S. M. Fuerstenberg, R. A. Zager, B. Torok-Storb. 1994. Kidney Int. 45: 48-57.). Exposure of HK-2 cells to H₂O₂ 1mM induced a significant increase in CAIII mRNA expression as early as 3 hours postincubation, with a maximal level observed at 6 hours posttreatment. Immunoblotting analyses showed an early and stable induction of CAIII from 6 hours postincubation with H₂O₂ (FIG. 10). Similarly, exposure of OK cells to H₂O₂ 0.3 mM was associated with an increased expression of CAIII from 6 hours postincubation (data not shown). These data demonstrate that PT cells rapidly increase their endogeneous expression of CAIII in response to oxidative conditions.

### 3. Discussion

Despite the physiological importance of PT cells and the clinical severity of the renal Fanconi syndrome, the cellular and metabolic outcomes of inherited PT dysfunction remain essentially unknown. Using mouse, human and cellular models, we show here that inherited PT dysfunction is associated with higher cell turnover and increased cellular response to oxidant damage, as well as a major upregulation of CAIII, an isozyme that was previously unknown in the kidney. The induction of CAIII in *Clcn5*^{*Y*/*-*} mice was restricted to the kidney, with no changes observed in the other CAIII -expressing organs. These findings were confirmed in a patient harbouring a missense mutation in *CLCN5* and another mouse model of PT dysfunction caused by the inactivation of megalin. Moreover the exposure of two distinct PT cell lines to oxidant conditions caused a rapid and consistent response of CAIII.

Due to their intense reabsorptive activity involving active transport, the epithelial cells lining the PT are particularly vulnerable to injury. However, in contrast to brain and heart, the kidney can completely recover from an ischemic or toxic insult. Following cell death by necrosis and apoptosis, the surviving PT cells dedifferentiate and proliferate to eventually replace the injured epithelial cells and restore tubular integrity (Bonventre, J. V. 2003. J. Am. Soc. Nephrol. 14: S55-S61.). Our studies reveal that a similar process occurs as a response to a chronic injury, i.e. inherited renal Fanconi syndrome in mouse and man. The proliferative activity of PT cells, assessed using antibodies to cell proliferation-associated nuclear proteins PCNA and KI-67, was almost 4-fold increased, with the involvement of the G1 cell cycle kinase being suggested by the upregulated cyclin E. Furthermore, PT cells underwent dedifferentiation, as indicated by the expression of osteopontin and the mesodermal marker CAIII (see below). These modifications occurred at a time when no visible alterations in PT cell morphology nor changes in renal function were observed (Wang, S. S. 2000. Hum. Mol. Genet. 9: 2937-2945), and without change in the apoptotic rate. The growth and/or transcription factors that could be involved in this adaptative response remain to be defined. In particular, the lack of albumin endocytosis due to impaired receptor-mediated endocytosis in these models, which normally activates various signal transduction cascades in PT cells, may play a major role (Imai, E. et al 2004. Kidney Int. 66: 2085 - 2087.).

An important finding of this study is the demonstration of a significantly increased production of superoxide anion in the *Clcn5*^{*Y*/*-*} PT cells, with the parallel induction of SOD and thioredoxin, pointing to increased oxidative stress and solicitation of cell oxidative defences. Thioredoxin was also selectively increased in a human kidney with inactivating CIC-5 mutation. Increased ROS production, which depletes endogenous radical scavengers and causes cell oxidative damage, is involved in PT lesions induced by nephrotoxic compounds such as cisplatin and heavy metals (Percy, C., B. et al 2005. Adv. Chronic Kidney Dis. 12: 78-83.). Similarly, IV iron produces an oxidative stress that is associated with transient proteinuria and tubular damage (Agarwal, R. et al. 2004. Kidney Int. 65: 2279-2289.). Furthermore, Wilmer *et al.* have shown a significantly elevated level of oxidized glutathione in PT cells derived from patients with a renal Fanconi syndrome due to cystinosis (Wilmer, M. J. et al. 2005. Biochem. Biophys. Res. Commun. 337: 610-614.). Taken together, these data suggest that acquired and inherited dysfunctions of the PT are associated with a state of oxidative stress. Considering the importance of albumin endocytosis in the regulation of H₂O₂ production in PT cells (Imai, E, et al 2004. Kidney Int. 66: 2085 - 2087.), it is tempting to hypothesize that the lack of albumin endocytosis in PT cells lacking CIC-5 may lead to unbalanced production of H₂O₂, that in turn could contribute to PT dysfunction.

Type III CA belongs to the family of zinc metallo-enzymes that reversibly hydrate CO₂, thus generating hydrogen and bicarbonate ions essential for acid-base homeostasis, respiration, ureagenesis, lipidogenesis, urinary acidification and bone resorption (Lindskog, S. 1997. Pharmacol. Ther. 74: 1-20.; Sly, W. S., P. Y. Hu. 1995. Annu. Rev. Biochem. 64: 375-401.). At least 15 different isoforms, with 11 catalytically active isozymes, have been described in the mammals, with distinct kinetic properties and tissue distribution. Subcellularly, four of the active CA isozymes are cytosolic (CAI, CAII, CAIII, and CAVII), four are membrane-bound (CAIV, CAIX, CAXII and CAXIV), two are mitochondrial (CA VA and VB), and one is a secretory isoform (CAVI) (Mori, K. et al. 1999. J. Biol. Chem. 274: 15701-15705.). Type II and IV CA represent the two main isozymes in the kidney, located in PT cells where they participate in H⁺ secretion and HCO₃⁻ reabsorption, as well as to NaCl homeostasis. CAII is present in the cytosol of the intercalated cells of the collecting duct, where it ensures net urinary acidification. The functional loss of CAII causes Guibaud-Vainsel disease, an inherited syndrome characterized by renal tubular acidosis, osteopetrosis, and cerebral calcifications (Sly, W. S., et al. 1985. N. Engl. J. Med. 313: 139-145.). Two other CA isozymes have been located in mouse kidney, i.e. CAXIII and CAXIV, but their specific role, as well as their interactions with CAII and CAIV in this organ, remain unknown (Lehtonen, J., B. et al, 2004. J. Biol. Chem. 279: 2719-2727., Mori, K., Y. et al. 1999. J. Biol. Chem. 274: 15701-15705.).

Type III CA is distinguishable from the other CA isozymes by several features, particularly its resistance to sulfonamide inhibitors and its low CO₂ hydration ability which represents -2% of CAII activity (Jewell, D. A. et al. , 1991. Biochemistry 30: 1484-1490.). Its lower catalytic turnover is in part explained by the replacement of a histidine by a lysine at residue 64, which is not efficient for proton transfer during catalysis. In addition, the phenyl side chain of Phe 198 (instead of Leu in CAII) causes a steric constriction of the CAIII active site, which may also explain the lower catalytic activity and resistance to acetazolamide (Duda, D. M. et al. 2005. Biochemistry. 44: 10046-10053., 25). Although CAIII is abundantly expressed in the cytosol of skeletal muscle cells, adipocytes and hepatocytes, its function and regulation remain unclear (Kim, G., T et al. 2004. Mol. Cell Biol. 24: 9942-9947). In addition, CAll is an early mesodermal marker expressed in embryonic mouse notochord, that defines a subset of mesodermal cell types later during embryogenesis (Lyons, G. E., et al, Development. 111: 233-244.). Along with other genes encoding growth/transcription factors, that recapitulate the expression pattern seen during nephrogenesis (Bonventre, J. V. 2003. J. Am. Soc. Nephrol. 14: S55-S61). CAIII may thus represent a novel marker of cell dedifferentation associated with inherited PT dysfunction.

CAIII is known to function in an oxidizing environment (Cabiscol, E., R. L. Levine. 1995. J. Biol. Chem. 270: 14742-14747.), which is of interest when considering the oxidative stress evidenced in the mouse and human PT cells investigated here. Two reactive sulfhydryl groups of CAIII are rapidly S-thiolated by glutathione after *in vivo* and *in vitro* exposure to oxidative conditions, and dethiolated by enzymatic reactions (glutaredoxin and thioredoxin-like) (Chai, Y. C et al. 1991. Arch. Biochem. Biophys. 284: 270-288.). CAIII also plays a protective role against H₂O₂-induced apoptosis, contrasting with the lack of effect of the CAII isoform (Raisanen, S. R., et al 1999. FASEB J. 13: 513-522.). Furthermore, NIH/3T3 cells overexpressing CAIII grow faster and are more resistant to cytotoxic concentrations of H₂O₂ than control cells. Thus, by analogy with acute (ischemia-reperfusion) and chronic (aging) injuries (Cabiscol, E., R. L. Levine. 1995. J. Biol. Chem. 270: 14742-14747., Eaton, P., et al. 2003. J. Am. Soc. Nephrol. 14: S290-S296.), CAIII may function as an oxyradical scavenger, protecting PT cells from the oxidative stress induced by chronic dysfunction. Of note, Kim et al. have postulated that CAIII may have evolved into a percarbonic acid anhydrase, which would mediate H₂O₂ + CO₂ ↔ H₂CO₄ (Richardson, D.E., et al. 2003. Free Radic. Biol. Med. 35: 1538-1550, and Kim, G et al. 2004. Mol. Cell Biol. 24: 9942-9947.).

What could be the regulator of CAIII induction associated with PT dysfunction? The concentration of CAIII in rat male liver was found to be 30 times greater than that in females, with a marked reduction after castration, suggesting regulation by androgens (Carter, N., et al 2001. Ups J. Med. Sci. 106: 67-76.). However, comparative real-time RT-PCR studies in mouse kidney did not find any significant difference in CAIII mRNA expression between 15-week-old males and females (*n*=*5*, data not shown). Thyroidectomy is known to increase CAIII concentration in rat muscle (Jeffery, S., J. Histochem. Cytochem. 35: 663-668.), which could be relevant when considering that CIC-5 is highly expressed in wild-type mouse thyroid (van den Hove, M.-F. et al 2006. Endocrinology. 147: 1287-1296.). However, mice lacking CIC-5 develop a euthyoid goiter, without alterations in circulating T4 and TSH levels. In contrast, preliminary data suggest that the transcription factor hepatocyte nuclear factor 1alpha (HNF1 α), which is expressed in liver, pancreas, intestine and kidney, may regulate the transcription of CAIII in PT cells. *In silico* analysis of the promoter of the *Car3* gene revealed several potential HNF1 binding sites, and mice lacking HNF1α, which show a severe PT dysfunction reflected by polyuria, glucosuria, aminoaciduria and phosphaturia, are characterized by a decreased renal expression of CAIII (F. Jouret, K. Parreira and O. Devuyst, unpublished observations).

Finally, the selective detection of CAIII in the urine of mice lacking CIC-5 and other congenital and acquired models of PT dysfunction (F. Jouret and O. Devuyst, unpublished data) suggests that the urinary excretion of CAIII may be a useful biomarker of renal Fanconi syndrome. As compared with traditional markers, such as low-molecular-weight proteins (e.g. β2-microglobulin), which are produced outside the kidney, filtered by the glomerulus but not reabsorbed due to defective PT endocytosis, or PT cell components (such as N-acetyl-beta-glucosaminidase, NAG) that appear in the urine in case of structural alterations, CAlll in the urine may directly reflect a state of cellular dysfunction, without changes in renal function or morphology. Furthermore, the upregulation of CAlll is organ- and segment-specific, and clearly conserved in mouse and man.

In conclusion, we report on CAIII, a novel kidney CA isozyme that is specifically induced in scattered mouse and human PT cells, where it may participate to the cellular response against oxidative damage associated with chronic PT dysfunction.

## Claims

1. A method for determining a condition related to dysfunction of the RPT in a subject comprising detecting the presence of type III Carbonic Anhydrase, CA-III, in a urine sample of said subject.

2. Method according to claim 1 comprising the steps of:
(i) obtaining a urine sample from a subject;
(ii) measuring the concentration of CA-III in the sample;
(iii) comparing the concentration of CA-III in the sample with the concentration of CA-III in a healthy subject; and
(vi) determining a condition related to dysfunction of the PRT when the concentration of CA-III in the sample is at least 10% different from that measured in a sample from a healthy subject.

3. Method according to claim 1 comprising the steps of:
(i) obtaining a urine sample from a subject;
(ii) measuring the concentration of CA-III in the sample;
(iii) determining a condition related to dysfunction of the PRT when detectable CA-III is present in the sample or concentration of CA-III in the sample is greater than or equal to a threshold concentration.

4. Method according to claim 3 wherein said threshold value is between 1 pM and 1 mM.

5. Method according to claim 1 comprising the steps of:
(i) obtaining a urine sample from a subject;
(ii) detecting the presence of CA-III the sample;
(vi) determining a condition related to dysfunction of the PRT when CA-III is detected in the sample.

6. Method for monitoring the progress of a condition related to dysfunction of the PRT in a subject by monitoring the concentration of CA-III in two or more urine samples taken at different intervals.

7. Method according to claim 6, comprising the steps of:
(i) obtaining two or more urine samples from a subject, taken at different time intervals;
(ii) measuring the concentration of CA-III each sample;
(iii) determining the progress of a condition related to dysfunction of the PRT by comparing the concentrations of CA-III in the measured samples over time.

8. Method for monitoring the efficacy of a treatment of a condition related to dysfunction of the PRT in a subject by detecting the level of CA-III in a urine sample taken before, after and optionally during treatment.

9. Method according to claim 8, comprising the steps of:
(i) obtaining a pre-administration urine sample from a subject prior to administration of the treatment;
(ii) measuring the concentration of CA-III in the pre-administration sample;
(iii) obtaining one or more post-administration urine samples from the subject;
(iv) measuring the concentration of CA-III the post-administration samples;
(v) comparing the concentration of CA-III in the pre-administration sample with the level of CA-III in the post-administration sample or samples; and
(vi) determining the efficacy of a treatment.

10. Method according to claim 9, further comprising the step of altering the treatment to improve the effect thereof.

11. Method according to any of claim 1 to 10, wherein the concentration or presence of CA-III in a sample is measured by using a CA-III specific probe.

12. Method according to claim 11, wherein said CA-III probe is an antibody directed against CA-III or a fragment thereof.

13. Method according to claim 12 wherein said antibody is a polyclonal antibody, monoclonal antibody, humanised or chimeric antibody, engineered antibody, or biologically functional antibody fragments sufficient for binding to CA-III.

14. Method according to claim 12, wherein said antibody is mouse monoclonal antibody clone 2CA-4.

15. Method according to any of claims 1 to 14 wherein said CA-III human CA-III, a polypeptide having the sequence represent by SEQ ID NO: 1, or a fragment thereof.

16. Method according to any of claims 11 to 15, wherein the concentration or presence of CA-III is measured using any of biochemical assay, immunoassay, surface plasmon resonance, fluorescence resonance energy transfer, bioluminescence resonance energy transfer or quenching.

17. Method according to any of claims 1 to 16, wherein said condition is Fanconi syndrome or Dent's disease.

18. Method according to any of claims 1 to 16, wherein said condition is nephrotoxicity.

19. Method according to claim 18, wherein said condition arises from ingestion or infusion of heavy metals, chemotherapy agents, toxic drugs, poisons, pollutants, toxins or after injection with an iodinated contrast dye.

20. Method according to any of claim 1 to 16, wherein said condition is as a result of a physical renal injury.

21. Method according to any of claim 1 to 16, wherein said condition is renal or kidney failure or dysfunction.

22. Method according to claim 21, wherein said condition is acute renal failure include sepsis, shock, trauma, kidney stones, kidney infection, drug toxicity, poisons or toxins, or after injection with an iodinated contrast dye.

23. Method according to claim 21, wherein said condition is chronic renal failure, long-standing hypertension, diabetes, congestive heart failure, lupus, or sickle cell anemia.

24. Method according to any of claim 1 to 16, wherein said condition is inherited or acquired.

25. Method according to any of claims 1 to 24, further comprising detecting the presence of proteins and sugar in the urine.

26. Use of CA-III as a urinary biomarker.

27. Use of CA-III for diagnosing a condition related to dysfunction of the PRT in a subject.

28. Device for determining a condition related to dysfunction of the PRT in a subject comprising means for determining the concentration and/or presence of CA-III in said urine sample.

29. Device according to claim 28, wherein said means comprise at least one CA-III specific probe.

30. Device according to claim 29, wherein said CA-III specific probe is as defined in any of claims 12 to 14.

31. Device according to any of claims 28 to 30 comprising a solid support whereby said CA-II is immobilised thereon.

32. Device according to claim 31 wherein said solid support has fluid capillary properties and comprises:
- a distal (3) and proximal end (2),
- a sample application zone (4) in the vicinity of the proximal end (2),
- a reaction zone (5) distal to the sample application zone (4),
- a detection zone (6) distal to the reaction zone (5),
- where the reaction zone (5) is disposed with CA-III probe labelled with detection agent, that can migrate towards the distal end (3) in a flow of fluid by capillary action,
- where the detection zone (6) comprises said immobilised CA-III probe that can capture CA-III.

33. Device according to claim 32 housed in a cartridge (20) watertight against urine, having an opening (21) to provide access to the application zone (4) in proximal end (2), and another opening (22) to enable reading of detection zone (6) close to the distal end (3).

34. Device according to claim 33, wherein said cartridge (20) is disposed with a sensor code (23) for communicating with a reading device.

35. A kit comprising a device according to any of claims 28 to 34 and a urine sample container and/or control standards comprising CA-III.


SEQUENCE LISTING

<110>  Université Catholique de Louvain

<120>  Method, device and kit for determining conditions related to a
       dysfunction of the renal proximal tubule

<130>  UCL-075-EP-Prio

<140>  EP 07447011.3
<141>  2007-02-12

<160>  48

<170>  PatentIn version 3.3

<210>  1
<211>  260
<212>  PRT
<213>  Homo sapiens

<400>  1

Met Ala Lys Glu Trp Gly Tyr Ala Ser His Asn Gly Pro Asp His Trp
1               5                   10                  15

His Glu Leu Phe Pro Asn Ala Lys Gly Glu Asn Gln Ser Pro Val Glu
            20                  25                  30

Leu His Thr Lys Asp Ile Arg His Asp Pro Ser Leu Gln Pro Trp Ser
        35                  40                  45

Val Ser Tyr Asp Gly Gly Ser Ala Lys Thr Ile Leu Asn Asn Gly Lys
    50                  55                  60

Thr Cys Arg Val Val Phe Asp Asp Thr Tyr Asp Arg Ser Met Leu Arg
65                  70                  75                  80

Gly Gly Pro Leu Pro Gly Pro Tyr Arg Leu Arg Gln Phe His Leu His
                85                  90                  95

Trp Gly Ser Ser Asp Asp His Gly Ser Glu His Thr Val Asp Gly Val
            100                 105                 110

Lys Tyr Ala Ala Glu Leu His Leu Val His Trp Asn Pro Lys Tyr Asn
        115                 120                 125

Thr Phe Lys Glu Ala Leu Lys Gln Arg Asp Gly Ile Ala Val Ile Gly
    130                 135                 140

Ile Phe Leu Lys Ile Gly His Glu Asn Gly Glu Phe Gln Ile Phe Leu
145                 150                 155                 160

Asp Ala Leu Asp Lys Ile Lys Thr Lys Gly Lys Glu Ala Pro Phe Thr
                165                 170                 175

Lys Phe Asp Pro Ser Cys Leu Phe Pro Ala Cys Arg Asp Tyr Trp Thr

180                 185                 190

Tyr Gln Gly Ser Phe Thr Thr Pro Pro Cys Glu Glu Cys Ile Val Trp
        195                 200                 205

Leu Leu Leu Lys Glu Pro Met Thr Val Ser Ser Asp Gln Met Ala Lys
    210                 215                 220

Leu Arg Ser Leu Leu Ser Ser Ala Glu Asn Glu Pro Pro Val Pro Leu
225                 230                 235                 240

Val Ser Asn Trp Arg Pro Pro Gln Pro Ile Asn Asn Arg Val Val Arg
                245                 250                 255

Ala Ser Phe Lys
            260

<210>  2
<211>  17
<212>  DNA
<213>  Artificial sequence

<220>
<223>  AFLP primer/adapter

<400>  2
ctcgtagact gcgtacc                                                    17

<210>  3
<211>  18
<212>  DNA
<213>  Artificial sequence

<220>
<223>  AFLP primer/adapter

<400>  3
aattggtacg cagtctac                                                   18

<210>  4
<211>  16
<212>  DNA
<213>  Artificial sequence

<220>
<223>  AFLP primer/adapter

<400>  4
gacgatgagt cctgag                                                     16

<210>  5
<211>  14
<212>  DNA
<213>  Artificial sequence

<220>
<223>  AFLP primer/adapter

<400>  5

tactcaggac tcat                                                    14

<210>  6
<211>  16
<212>  DNA
<213>  Artificial sequence

<220>
<223>  AFLP primer/adapter

<400>  6
gactgcgtac caattc                                                  16

<210>  7
<211>  16
<212>  DNA
<213>  Artificial sequence

<220>
<223>  AFLP primer/adapter

<400>  7
gatgagtcct gagtaa                                                  16

<210>  8
<211>  18
<212>  DNA
<213>  Artificial sequence

<220>
<223>  AFLP primer/adapter

<400>  8
gactgcgtac caattcaa                                                18

<210>  9
<211>  18
<212>  DNA
<213>  Artificial sequence

<220>
<223>  AFLP primer/adapter

<400>  9
gactgcgtac caattcac                                                18

<210>  10
<211>  18
<212>  DNA
<213>  Artificial sequence

<220>
<223>  AFLP primer/adapter

<400>  10
gactgcgtac caattcag                                                18

<210>  11
<211>  18
<212>  DNA
<213>  Artificial sequence

<220>
<223>  AFLP primer/adapter

<400>  11
gactgcgtac caattcat                                                    18


<210>  12
<211>  18
<212>  DNA
<213>  Artificial sequence

<220>
<223>  AFLP primer/adapter

<400>  12
gatgagtcct gagtaaaa                                                    18


<210>  13
<211>  18
<212>  DNA
<213>  Artificial sequence

<220>
<223>  AFLP primer/adapter

<400>  13
gatgagtcct gagtaaac                                                    18


<210>  14
<211>  18
<212>  DNA
<213>  Artificial sequence

<220>
<223>  AFLP primer/adapter

<400>  14
gatgagtcct gagtaaat                                                    18


<210>  15
<211>  20
<212>  DNA
<213>  Artificial sequence

<220>
<223>  PCR primer

<400>  15
cttgaagcac tgcattccat                                                  20


<210>  16
<211>  20
<212>  DNA
<213>  Artificial sequence

<220>
<223>  PCR primer

<400>  16
cttgatgccc tggacaaaat                                                  20


<210>  17

<211>  20
<212>  DNA
<213>  Artificial sequence

<220>
<223>  PCR primer

<400>  17
ttggaatccc agaacaggag                                                    20


<210>  18
<211>  20
<212>  DNA
<213>  Artificial sequence

<220>
<223>  PCR primer

<400>  18
tgcaaaggta gaggctccat                                                    20


<210>  19
<211>  20
<212>  DNA
<213>  Artificial sequence

<220>
<223>  PCR primer

<400>  19
tccaatcgtc cctacagtcg                                                    20


<210>  20
<211>  20
<212>  DNA
<213>  Artificial sequence

<220>
<223>  PCR primer

<400>  20
catggtctgg gacttctgga                                                    20


<210>  21
<211>  20
<212>  DNA
<213>  Artificial sequence

<220>
<223>  PCR primer

<400>  21
gggttccacg tccatcagta                                                    20


<210>  22
<211>  20
<212>  DNA
<213>  Artificial sequence

<220>
<223>  PCR primer

<400>  22

tgatcaagcc cttcttccat                                                    20


<210>  23
<211>  20
<212>  DNA
<213>  Artificial sequence

<220>
<223>  PCR primer

<400>  23
tgcaccacca actgcttagc                                                    20


<210>  24
<211>  20
<212>  DNA
<213>  Artificial sequence

<220>
<223>  PCR primer

<400>  24
acattgtggc cctctgtgtg                                                    20


<210>  25
<211>  20
<212>  DNA
<213>  Artificial sequence

<220>
<223>  PCR primer

<400>  25
cacgatccag gtcacacatt                                                    20


<210>  26
<211>  20
<212>  DNA
<213>  Artificial sequence

<220>
<223>  PCR primer

<400>  26
gagccgtggt aggtccaata                                                    20


<210>  27
<211>  20
<212>  DNA
<213>  Artificial sequence

<220>
<223>  PCR primer

<400>  27
attgccaagc tctccacttg                                                    20


<210>  28
<211>  19
<212>  DNA
<213>  Artificial sequence

<220>
<223>  PCR primer

<400>  28
caggtaggcc agagcaagt                                                    19


<210>  29
<211>  20
<212>  DNA
<213>  Artificial sequence

<220>
<223>  PCR primer

<400>  29
cgctcttcat gtgagaggtg                                                   20


<210>  30
<211>  20
<212>  DNA
<213>  Artificial sequence

<220>
<223>  PCR primer

<400>  30
gactgcctct ccatctgcat                                                   20


<210>  31
<211>  20
<212>  DNA
<213>  Artificial sequence

<220>
<223>  PCR primer

<400>  31
cagtcacatt gcccaggtct                                                   20


<210>  32
<211>  20
<212>  DNA
<213>  Artificial sequence

<220>
<223>  PCR primer

<400>  32
cccacctttt gacccttttt                                                   20


<210>  33
<211>  20
<212>  DNA
<213>  Artificial sequence

<220>
<223>  PCR primer

<400>  33
ggatgcaggg atgatgttct                                                   20


<210>  34

<211>  20
<212>  DNA
<213>  Artificial sequence

<220>
<223>  PCR primer

<400>  34
ttatgtcccc cgttgactga                                                    20


<210>  35
<211>  19
<212>  DNA
<213>  Artificial sequence

<220>
<223>  PCR primer

<400>  35
ccctggatgg cacttacag                                                     19


<210>  36
<211>  19
<212>  DNA
<213>  Artificial sequence

<220>
<223>  PCR primer

<400>  36
gccgggacta ctggaccta                                                     19


<210>  37
<211>  20
<212>  DNA
<213>  Artificial sequence

<220>
<223>  PCR primer

<400>  37
acgtctcttt ggtgcagctc                                                    20


<210>  38
<211>  20
<212>  DNA
<213>  Artificial sequence

<220>
<223>  PCR primer

<400>  38
atggccgagg tgatagtgtg                                                    20


<210>  39
<211>  20
<212>  DNA
<213>  Artificial sequence

<220>
<223>  PCR primer

<400>  39

tggctcattt tgaccgagag                                                    20

<210>  40
<211>  18
<212>  DNA
<213>  Artificial sequence

<220>
<223>  PCR primer

<400>  40
tcagccacgt ggtgtggg                                                      18

<210>  41
<211>  20
<212>  DNA
<213>  Artificial sequence

<220>
<223>  PCR primer

<400>  41
ggggctctcc agaacatcat                                                    20

<210>  42
<211>  24
<212>  DNA
<213>  Artificial sequence

<220>
<223>  PCR primer

<400>  42
cagctttccc aaaatcccca catt                                               24

<210>  43
<211>  19
<212>  DNA
<213>  Artificial sequence

<220>
<223>  PCR primer

<400>  43
cgttctcagc actggagag                                                     19

<210>  44
<211>  20
<212>  DNA
<213>  Artificial sequence

<220>
<223>  PCR primer

<400>  44
gcgttatctt cggcccttag                                                    20

<210>  45
<211>  20
<212>  DNA
<213>  Artificial sequence

<220>
<223>  PCR primer

<400>  45
gatggccttg tatgcaccat                                                    20


<210>  46
<211>  20
<212>  DNA
<213>  Artificial sequence

<220>
<223>  PCR primer

<400>  46
gcgatgggag tcttctttcc                                                    20


<210>  47
<211>  22
<212>  DNA
<213>  Artificial sequence

<220>
<223>  PCR primer

<400>  47
tggaatgttg gcatgcattt ga                                                 22


<210>  48
<211>  19
<212>  DNA
<213>  Artificial sequence

<220>
<223>  PCR primer

<400>  48
tctagacggc aggtcaggt                                                     19